# EUROPEAN PATENT SPECIFICATION

(11) **EP 4 288 025 B1**
(45) Date of publication and mention of the grant of the patent: **09.04.2025**
(21) Application number: 22712443.5
(22) Date of filing: 04.02.2022
(51) Int. Cl.: A61K 8/34, A61K 8/64, A61K 8/67, A61K 8/9789, A61K 38/19, A61Q 19/00, A61Q 19/08

(54) **COMPOSITION AND METHOD OF NON-THERAPEUTIC EXFOLIATION OF THE EPIDERMIS**
ZUSAMMENSETZUNG UND VERFAHREN ZUR NICHT-THERAPEUTISCHEN EXFOLIATION DER EPIDERMIS
COMPOSITION ET MÉTHODE D'EXFOLIATION NON-THÉRAPEUTIQUE DE L'ÉPIDERME

(30) Priority: 04.02.2021 PL 43686921
(43) Date of publication of application: 13.12.2023
(73) Proprietor: URGO RECHERCHE INNOVATION ET DEVELOPPEMENT, 21300 Chenôve (FR)
(72) Inventor: PAWLAK, Sandra, 05-509 Józefoslaw (PL); NACZELNIK, Izabela, 05-816 Michalowice-Wies (PL)
(74) Representative: Patpol Kancelaria Patentowa Sp. z o.o.
(86) International application number: PCT/IB2022/050974
(87) International publication number: WO 2022/167984

(56) References cited:
- WO-A1-2018/232527
- US-A1- 2017 157 000
- US-A1- 2019 159 991
- US-B1- 6 589 540

## Description

### TECHNICAL FIELD

The subject of the invention is a hydroalcoholic composition comprising retinol and a method of non-therapeutic exfoliation of the epidermis, in particular performed by a skilled professional, e.g. in a beauty salon.

### BACKGROUND ART

Retinoids are a class of compounds that are natural or synthetic analogs of vitamin A. Vitamin A and its derivatives (including retinol) are lipophilic substances used in humans, including in the treatment of skin diseases such as rosacea, psoriasis, inflammation of the hair follicles, etc. Retinoids penetrate the calloused layer of the epidermis to a varying degree, and this process also depends on the formulation in which they are applied to the skin. There are four groups of high affinity receptors in human keratinocytes, including for retinol, and this compound stimulates the proliferation of these cells, reduces transepidermal water loss, and inhibits the activity of metalloproteinases that degrade extracellular matrix proteins. In the outer layers of the epidermis, retinol weakens the connections between epidermal cells and facilitates exfoliation. Due to this, retinol, as well as other vitamin A derivatives, are also used in care and cosmetic preparations, including anti-aging and anti-wrinkle preparations, as well as brightening the skin and improving its color. These substances are also used to counteract acne lesions and problems with uneven exfoliation of the epidermis.

A known problem, however, is the potential irritant effect of this group of compounds. The most common symptom is local skin irritation, associated with redness and visible exfoliation of the outer layers of the epidermis. Applying the composition with retinol to the skin can result in inflammation, allergic reactions, exfoliation of the skin, severe redness, discomfort such as stinging and itching, and increased sensitivity to UV radiation.

Various approaches are known in the art to reduce or counter these adverse effects. For this purpose, it is often recommended to simultaneously use skin moisturizing and oiling ingredients. Numerous solutions are known in the art, including the use of less irritating derivatives, encapsulating the active compound in microspheres or other carriers, or placing it in compositions with a high content of lipid compounds, including fatty emulsions, often additionally enriched with moisturizing and soothing ingredients. Formulations of retinol based on hydrogels, hydroalcoholic compositions etc. are known, but their use is associated with a potentially higher risk of adverse reactions, particularly at the site of administration. Therefore, particularly when using higher concentrations of retinol, rather emulsion-based formulations are chosen, often with the need for additional use of more complex (e.g., liposomal) delivery systems (see, e.g., Ferreira, R., Napoli, J., Enver, T. et al. Advances and challenges in retinoid delivery systems in regenerative and therapeutic medicine. Nat Commun 11, 4265, 2020). The concentrations of retinol or analogs used in cosmetic formulations are usually in the range of up to 0.05 wt.% (retinol equivalent) in personal care balms and lotions and up to 0.3 wt.% in hand and face creams and washable products. In preparations for treatments performed under the supervision of a specialist (e.g. for intensive exfoliating treatments), higher concentrations of retinol and analogues are used (e.g. 4% by weight of retinol). However, due to the irritating potential, emulsion compositions are used more often, and in less frequently used gel or hydroalcoholic preparations, lower concentrations of retinol are usually used. Despite the use of additional soothing preparations, skin irritation is expected, as well as a period of one to several days when the person after the treatment may have to stay at home due to the intense and visible peeling of the skin.

The concentrations of retinol or analogs thereof used in cosmetic preparations are usually in the range of up to 0.05% wt (retinol equivalent) in personal care balms and lotions and up to 0.3% wt in hand and face creams and washable products. In preparations for treatments performed under the supervision of a specialist (e.g. for intensive exfoliating treatments), higher concentrations of retinol and analogues are used (e.g. 4% by weight of retinol). However, due to the irritating potential, emulsion compositions are used more often, and in less frequently used gel or hydroalcoholic preparations, lower concentrations of retinol are usually used. Despite the use of additional soothing preparations, skin irritation is expected, as well as a period of one to several days when the person after the treatment may have to stay at home due to the intense and visible exfoliation of the skin.

Compositions comprising retinol or its derivatives are described, inter alia, in applications no. US6589540,

WO2016118281, US 2013/0310355, WO2014116653. FR 2 894 820 describes the use of soothing ingredients such as allantoin, EDTA in combination with retinoids. In the international application no. WO2005079775 idebenone and derivatives thereof have been used to alleviate the irritation associated with the use of retinoids. In turn, in the international application no. WO2005039532, retinoids were used in the composition of an oil microemulsion with hyaluronic acid. International application WO2017116908 describes the use of chitosan in a composition for the purpose of reducing irritant potential (such as associated with exposure to retinoids, e.g. retinol 0.01% - 1%). The application WO2015082659 discloses lipid microcapsules as carriers increasing the safety of retinol use.

In practice, retinol esters (such as palmitate) are often used in cosmetic preparations, for which the anti-aging potential is much weaker than that of retinol, yet they exhibit greater stability and provide an antioxidant effect. Retinol itself, while potentially more effective, is a reactive and much less stable ingredient. Its oxidation may result in the formation of potentially harmful decomposition products, and may also lead to isomerization or polymerization, which reduces the availability of active retinol from the formulation. Various solutions exist in the prior art to increase the stability, such as the use of additional antioxidants or chelating compounds, which, however, are not very effective in oil-in-water emulsions. Better stability effects have been obtained with water-in-oil emulsions, however, it is still necessary to use stabilizing ingredients (such as antioxidants) which can increase the irritant potential of the composition (see, inter alia, WO2003105806). Hence, very complex carrier compositions are used to stabilize retinol, e.g. providing multilayer crystal structures (WO2020225232).

Thus, there is an ongoing need for new retinol compositions that maintain its stability and efficacy while minimizing irritation potential.

The present inventors have found that when combining retinol with a peptide with a sequence corresponding to human TGF-beta 2 expressed in cells of the *Nicotiana benthamiana* plant (commercially available under the name Reneseed; INCI name: Nicotiana Benthamiana Hexapeptide-40 sh-Polypeptide-76), it is possible to achieve a lower skin irritation potential despite the administration of both components in a hydroalcoholic formulation. Surprisingly, despite the use of a relatively high concentration of retinol, a much less painful process of exfoliation was also observed. One of the expected effects of treating the skin with retinol is flaky exfoliation of the epidermis, which is usually a temporary, but nevertheless inconvenient, aesthetic effect of such a procedure, in some cases eliminating one from social life for a few days. When the retinol was combined in the composition with the peptide indicated above, the exfoliation of the epidermis took place in smaller fragments (without the effect of breaking the mask), was less visible and more spread over time.

The active ingredient, having INCI name of Nicotiana Benthamiana Hexapeptide-40 sh-Polypeptide-76 are two polypeptides connected by a single disulfide bridge. It is a fusion peptide from the combination of a peptide having INCI name of Hexapeptide-40 (the hexapeptide consisting of histidines) with a single-chain recombinant peptide obtained by transient expression of a synthetic copy identical to the human gene, encoding human TGF beta 2, in *Nicotiana benthamiana* cells. This component is described, inter alia, in the application EP3162357; it is also commercially available under the name Reneseed^{™}. According to the manufacturer's data, this ingredient has an anti-aging effect, analogous to retinol.

The subject of the present invention is therefore a hydroalcoholic based composition, comprising retinol, wherein the composition comprises retinol in a concentration of 3-7 wt.%, and an ingredient having INCI name of *Nicotiana Benthamiana* Hexapeptide-40 sh-Polypeptide-76.

Preferably, the composition comprises retinol in a concentration of 3.5 - 5 wt.%.

Preferably, the composition comprises *Nicotiana Benthamiana* Hexapeptide-40 sh-Polypeptide-76 in an amount of 0.000002 - 0.00003 wt.%.

The invention also relates to a method of non-therapeutic exfoliation of the outer layers of the epidermis, which comprises the steps of:
a) optional skin cleansing or make-up removal;
b) optional degreasing of the skin, optionally also chemical or mechanical peeling;
c) applying a preparation comprising antioxidant ingredients, preferably comprising vitamin C and ferulic acid;
d) protecting sensitive areas such as eye corners, nostrils, eyebrow lines, corners of the mouth, e.g. by applying a fatty substance such as petroleum jelly;
e) applying the composition according to the invention,
wherein the composition applied in step e) is not washed from the skin for a period of at least 6-10 hours.

Preferably, the method is performed by a skilled professional.

In a preferred embodiment, the composition applied in step e) is not washed from the skin for a period of 6-10 hours.

Preferably, the method comprises the step of a) cleansing the skin or removing make-up and / or the step of b) degreasing the skin, optionally also carrying out a chemical or mechanical peeling.

As used herein, the term hydroalcoholic formulation or composition or hydroalcoholic based composition means a composition comprising the active ingredient(s) based on a non-aromatic monohydric alcohol and water, the composition being suitable for non-therapeutic application to the surface of the skin.

A skilled professional, as defined in this document, is a skilled professional who performs professional non-therapeutic skin care treatments, such as a cosmetologist. The method defined herein uses a preparation with a high concentration of retinol (in the range of 3-7%), which aims to induce intense exfoliation of the epidermis for aesthetic purposes (younger appearance of the skin, more radiant skin with a more uniform color). Preparations with such an effect and such concentration of retinol are intended for use under the supervision of a specialist (e.g. in a beauty salon). The present invention relates only to an aesthetic, and therefore non-therapeutic, method of exfoliating the outer layers of the epidermis. Therapeutic methods and treatments, such as those performed by dermatologists for the treatment of skin diseases, are not within the scope of the present invention.

Oily skin is understood as a type of skin characterized by increased secretion of sebum. This type of skin is often visibly shiny and may have an earthy color; often coexists with problems with the visible openings of the sebaceous glands or comedones.

Dry / sensitive skin is a skin type characterized by a lower water content in the epidermis (<12% water in the stratum corneum). This type of skin is usually associated with increased sensitivity, a greater risk of irritation, frequent reactions of redness, itching, etc. Often the cause is deficiencies in the protective lipid layer in the epidermis.

Normal skin is characterized by a proper water content in the epidermis (basal layer approx. 70-50%, spinous layer approx. 50-40%, granular layer approx. 40-20%, stratum corneum approx. 12-15%.

Skin lightening ingredients within the meaning of the present invention are non-therapeutic active ingredients which, when applied to the skin, have a lightening effect, smooth the skin tone, etc. Examples of skin lightening ingredients are hydroquinone, kojic acid, arbutin, plant extracts (e.g. from *Morus alba, Citrus limonum, Crocus sativus*). Antioxidant ingredients (such as vitamin C) can also provide or support this effect. Ingredients protecting against UV influence include: substances classified as UV filters in Regulation (EC) No 1223/2009 of the European Parliament and of the Council of 30 November 2009 on cosmetic products, such as zinc oxide, titanium dioxide, Ethylhexyl triazone and others. Antioxidant ingredients are any ingredients with an antioxidant effect (such as vitamin C, ferulic acid, plant extracts, etc.). A person skilled in the art, without undue experimentation, will be able to identify ingredients with appropriate action that can be used in cosmetic compositions.

The present solution allows the use of high concentrations of retinol, in the range of 3-7%, reducing the risk of irritation regardless of the skin type, in particular also when used in a hydroalcoholic composition.

When using the composition according to the present invention, a very fast aesthetic effect in the form of visual illumination of the skin is observed. Despite the use of a hydroalcoholic matrix, after applying the product to the face and evaporating the solvents, no immediate skin irritation reaction is observed. The observed exfoliation of the epidermis is in the form of detachment of smaller fragments ("snow fling" effect) and not the flaky flaking that would be expected from a treatment with a higher concentration of retinol. The effect when using the composition according to the invention is therefore much less painful for the person using the composition according to the invention, compared to the compositions known from the prior art, the use of which often required staying at home for several days after administration of the composition due to the visible flaking of the skin.

Interestingly, the technical effect of using the composition according to the invention was similar regardless of the skin type (no significant differences were observed between oily skin and dry and sensitive skin). It is particularly important that the expected irritating effect was not observed, also when used on sensitive skin (e.g. dry skin, easily reacting with redness and inflammation).

The examples show exemplary compositions of the compositions according to the invention. This composition may be based on any hydroalcoholic base known in the art, and may also comprise any functional ingredients known in the art (such as thickeners, solvents, stabilizers, preservatives, antioxidant ingredients or the like). A person skilled in the art will select the functional components of such a composition without undue experimentation. The composition may also comprise optional additional active ingredients known in the art, e.g. plant extracts. Basically, the composition according to the invention is in any variant based on a hydroalcoholic base and comprises 3 - 7 wt.% of retinol and an ingredient having an INCI name of Nicotiana Benthamiana Hexapeptide-40 sh-Polypeptide-76.

The composition according to the invention may comprise the following ingredients:
Water: up to 100 wt.%
Alcohol (ethanol): 10-30 wt.%
Optional additional solvent or solvents, e.g. PEG-8, butylene glycol, isopropyl alcohol, diethyl ether: 10-40 wt.%
Optional emulsifier or surfactant ingredient, e.g. ethoxylated hydrogenated castor oil, polysorbate: 10-40 wt.%
Optional consistency-improving ingredients such as glycerin, isopropyl alcohol: 1-15 wt.%
Optional antioxidant and composition protecting ingredients such as BHT: e.g. up to 2 wt.%
Retinol; 3-7 wt.%
Nicotiana Benthamiana Hexapeptide-40 sh-Polypeptide-76 - preferably 0.000002 - 0.00003 wt.%. This ingredient may be prepared in advance in an aqueous solution (or water with another solvent, e.g. 40-60% butylene glycol) with an active ingredient concentration of e.g. 0.00009-0.00012 wt.% in solution, and the solution thus obtained may be added to the prepared composition of the invention (e.g. in an amount of 1-2 wt.% of the solution, or 1.5-2 wt.% of the solution).

Optionally, the composition may comprise other active ingredients, e.g. moisturizing (such as lactic acid, e.g. bizabolol, e.g. 0.1-1 wt.%) or additional epidermal exfoliating ingredients (e.g. lactate, e.g. sodium lactate, e.g. 0.01-1 wt.%).

The composition of the invention can be prepared in a manner known in the art, e.g. by mixing the ingredients (e.g. with a mechanical stirrer) at room temperature and adding water to 100%. Optionally, the active ingredient, e.g., Nicotiana Benthamiana Hexapeptide-40 sh-Polypeptide-76, may be pre-formulated in an aqueous solution and this pre-formulated solution may be added to the composition. It is also possible to mix the hydrophilic and lipophilic ingredients separately and then mix the two phases thus obtained.

The method of preparing the composition as well as the functional ingredients and optional additional active ingredients can therefore be standard and are known to the person skilled in the art. The essence of the solution according to the invention is, however, the possibility of combining relatively high concentrations of retinol (3-7 wt.%) with the above-mentioned peptide, in a hydroalcoholic formulation, with a low irritating potential combined with a very good non-therapeutic exfoliation efficiency, while providing better aesthetic effect, with less visible flaking, which is a clear advantage for the person applying the composition.

The composition according to the invention can also be used, for example, in a non-therapeutic exfoliation treatment performed by a skilled professional (e.g. a cosmetologist). Such a method of non-therapeutic epidermal exfoliation performed by a skilled professional is also an object of the present invention. The method may involve the addition of other formulations (e.g., a pre-formulation, an example of which is given in Table 6 below). Such a pre-formulation may comprise antioxidant ingredients, preferably vitamin C and ferulic acid, which are known in the art.

Optionally, the method may use additional preparations, e.g. a makeup remover or degreaser (e.g. standardly used in the art), peeling (for example, chemical or mechanical peels known in the art), as well as optional soothing preparations (e.g., oiling, comprising known soothing ingredients, including vitamin E) or preparations known in the art to protect the skin against UV radiation.

### Brief description of the drawings

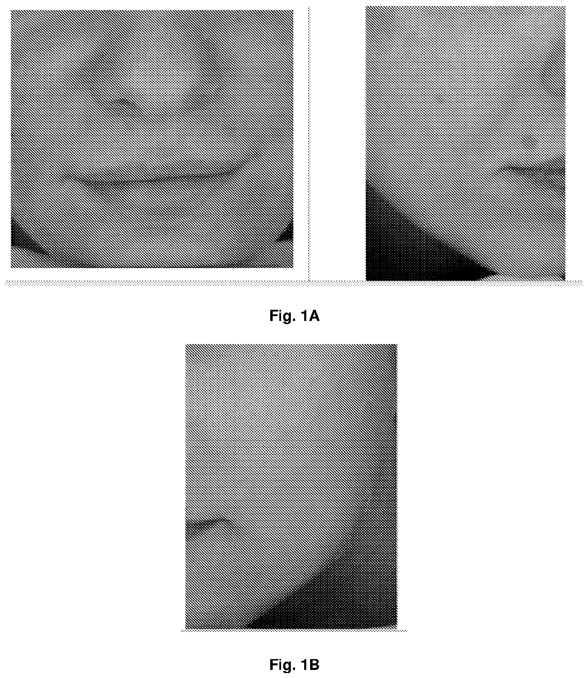

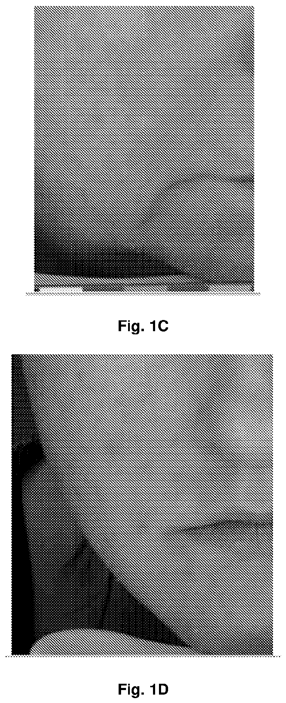

Fig. 1 shows exemplary photos of the skin condition and exfoliation after one treatment with the composition of the invention. Fig. 1A - group 1, normal and combination skin; Fig. 1B - group 2, normal and combination skin, additional anti-wrinkle and antioxidant care; Fig. 1C - group 4, sensitive/dry skin, additional anti-wrinkle and antioxidant care; Fig. 1D - group 5, thick skin with imperfections, oily skin.

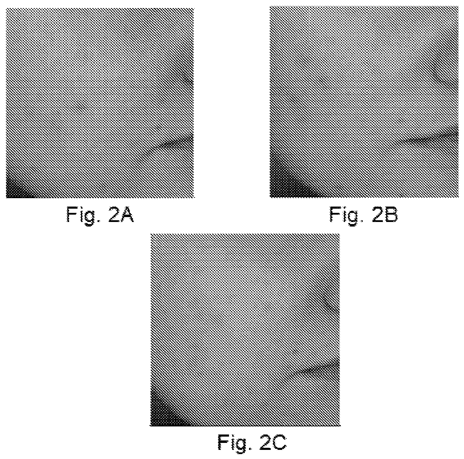

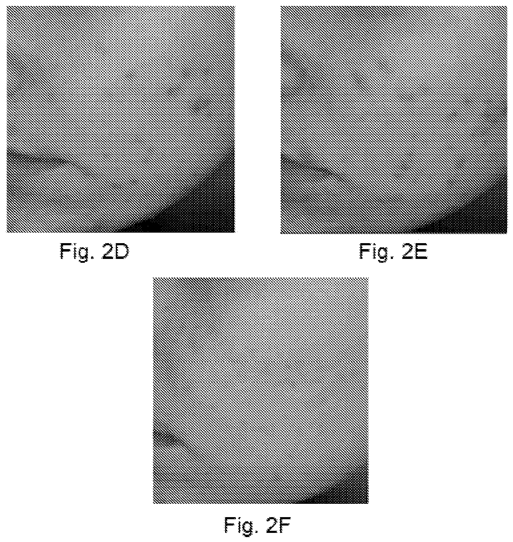

Fig. 2 shows exemplary photos of changes in the appearance of the skin surface of two volunteers using the composition according to the invention. Fig. 2A, appearance of the skin surface of volunteer 1/SS before the first treatment; Fig. 2B - appearance of the skin surface of volunteer 1/SS after the first treatment; Fig. 2C - appearance of the skin surface of volunteer 1/SS after the third treatment; Fig. 2D - appearance of the skin surface of volunteer 5/WP before the first treatment; Fig. 2E - appearance of the skin surface of volunteer 5/WP after the first treatment; Fig 2F - appearance of the skin surface of volunteer 5/WP after the third treatment.

### EXAMPLES

### Example 1. Preparation comprising retinol and a peptide mimicking TGF beta 2 fragment obtained from expression in plant cells.

The composition of ingredients indicated in Table 1 was prepared by mechanical mixing of the ingredients at room temperature, with water added to 100%. The obtained preparation was in the form of a colorless liquid with a pH in the range of 4.5 to 5.0 (at a temperature of 20 °C). The preparation was subjected to stability tests involving storage for 1, 2, 3 and 6 months at room temperature (20 - 25°C), 40°C and 5-8 °C. The preparation remained stable with a shelf life of 36 months.

**Table 1. Ingredients of an exemplary composition according to the invention**

| Substance | Contents (wt.%) |
|---|---|
| Water | up to 100% |
| PEG-8 | 20 |
| Castor oil PEG-35 | 16 |
| Ethanol | 13 |
| Butylene glycol | 11 |
| Polysorbate-20 | 4 |
| Retinol | 3.96 |
| Lactic acid | 3.6 |
| Ethoxydiglycol | 2.6 |
| Isopropyl alcohol | 1.5 |
| *Vaccinium myrtillus* fruit extract | 0.9 |
| *Saccharum officinarium* extract | 0.4 |
| Tocopheryl acetate | 0.3 |
| BHT | 0.3 |
| *Citrus aurantium dulcis* fruit extract | 0.1 |
| *Citrus limon* fruit extract | 0.1 |
| Bizabolol | 0.1 |
| BHA | 0.08 |
| *Acer saccharum* extract | 0.05 |
| Sodium lactate | 0.01 |
| Nicotiana Benthamiana Hexapeptide-40 sh-Polypeptide-76 | 0.0000024 |

### Example 2. Preparation comprising retinol and a peptide mimicking TGF beta 2 fragment obtained from expression in plant cells.

**Table 2. Composition of the preparation**

| Substance | Contents (wt.%) |
|---|---|
| Water | up to 100% |
| PEG-8 | 20 |
| Castor oil PEG-35 | 16 |
| Ethanol | 20 |
| Butylene glycol | 11 |
| Polysorbate-20 | 4 |
| Retinol | 5 |
| Lactic acid | 4 |
| Ethoxydiglycol | 2.6 |
| Isopropyl alcohol | 1.5 |
| Nicotiana Benthamiana Hexapeptide-40 sh-Polypeptide-76 | 0.0000024 |

### Example 3. Preparation comprising retinol and a peptide mimicking TGF beta 2 fragment obtained from expression in plant cells.

**Table 3. Composition of the preparation**

| Substance | Contents (wt.%) |
|---|---|
| Water | up to 100% |
| PEG-8 | 20 |
| Castor oil PEG-35 | 16 |
| Ethanol | 20 |
| Butylene glycol | 20 |
| Polysorbate-20 | 4 |
| Retinol | 4 |
| Lactic acid | 4 |
| Ethoxydiglycol | 2.6 |
| Isopropyl alcohol | 1.5 |
| Nicotiana Benthamiana Hexapeptide-40 sh-Polypeptide-76 | 0.00003 |

### Example 4. Preparation comprising retinol and a peptide mimicking TGF beta 2 fragment obtained from expression in plant cells.

**Table 4. Composition of the preparation**

| Substance | Contents (wt.%) |
|---|---|
| Water | up to 100% |
| PEG-8 | 20 |
| Castor oil PEG-35 | 16 |
| Ethanol | 20 |
| Butylene glycol | 20 |
| Polysorbate-20 | 4 |
| Retinol | 4 |
| Lactic acid | 4 |
| Ethoxydiglycol | 2.6 |
| Isopropyl alcohol | 1.5 |
| *Citrus aurantium dulcis* fruit extract | 1 |
| Nicotiana Benthamiana Hexapeptide-40 sh-Polypeptide-76 | 0.000005 |

### Example 5. Preparation comprising retinol and a peptide mimicking TGF beta 2 fragment obtained from expression in plant cells.

**Table 5. Composition of the preparation**

| Substance | Contents (wt.%) |
|---|---|
| Water | up to 100% |
| PEG-8 | 20 |
| Castor oil PEG-35 | 16 |
| Ethanol | 13 |
| Butylene glycol | 11 |
| Polisorbat-20 | 4 |
| Retinol | 3 |
| Ethoxydiglycol | 2,6 |
| Isopropyl alcohol | 1,5 |
| *Vaccinium myrtillus* fruit extract | 4 |
| Tocopheryl acetate | 0,3 |
| BHT | 0,3 |
| *Citrus aurantium dulcis* fruit extract | 0,1 |
| *Citrus limon* fruit extract | 0,1 |
| Bizabolol | 0,1 |
| BHA | 0,08 |
| *Acer saccharum* extract | 0,05 |
| Sodium lactate | 0,01 |
| Nicotiana Benthamiana Hexapeptide-40 sh-Polypeptide-76 | 0,000002 |

### Example 6. A skin exfoliating treatment

### Test methodology:

The effectiveness of the preparation in accordance with Table 1 was tested under the supervision of a cosmetologist. The study involved volunteers who were long enough in the waiting period between other treatments that stimulate or exfoliate the skin. During the tests, they did not perform any other treatments that could affect the evaluation of the test effectiveness.

A group of 33 individuals participating in the tests (full series of treatments) was qualified for the study.

The volunteers were women aged 29-65.

Different types of skins were tested (normal / combination, oily, dry / sensitive, mature).

The tests lasted 3-4 months.

Some of the volunteers also received complementary products for home use.

Each test participant had at least 5 visits:
- a start-up visit
- 3 treatment visits
- control visit (2-4 days after the first treatment)
- a control visit, 3-4 weeks after the end of the treatment series.

The primary aim of the study was to evaluate the effectiveness of the treatment using the preparation according to Table 1w. The secondary goal analysis concerned:
- evaluation of the tolerance of preparations and its influence on the effect of the treatment
- comparisons of the effectiveness with an analogous treatment, but performed with the use of an emulsion comprising 4% retinol (without Nicotiana Benthamiana Hexapeptide-40 sh-Polypeptide-76)
- evaluation of the application properties of the treatment with the use of the preparation according to Table 1
- evaluation of the effect of the proposed treatment on the quality of life of the volunteers.

The preparation used in the treatment had the composition shown in Table 1 above. The pre-preparation has composition as shown in Table 6.

**Table 6. Composition of pre-preparation**

| Substance | Contents (wt.%) |
|---|---|
| Water | up to 100% |
| Ascorbyl glucoside | 10 |
| Ethanol | 8.8 |
| Lactic acid | 2.7 |
| Ethoxydiglycol | 2.6 |
| Sodium hydroxide | 2.6 |
| Isopropyl alcohol | 1.1 |
| Niacinamide | 1 |
| Peg-40 Hydrogenated Castor Oil | 1 |
| *Vaccinium myrtillus* fruit extract | 0.9 |
| Ferulic acid | 0.5 |
| Triethanolamine | 0.5 |
| *Saccharum officinarium* extract | 0.4 |
| Sodium benzoate | 0.3 |
| Tocopheryl acetate | 0.3 |
| Panthenol | 0.2 |
| Xanthan gum | 0.2 |
| Potassium Sorbate | 0.2 |
| *Citrus aurantium dulcis* fruit extract | 0.1 |
| *Citrus limon* fruit extract | 0.1 |
| Sodium hyaluronate | 0.05 |
| EDTA disodium | 0.05 |
| *Acer saccharum* extract | 0.05 |
| Biosaccharide gum-2 | 0.03 |

The effectiveness of the treatment was assessed on the basis of the assessment of the person performing the treatment and the person who underwent the treatment.

The group that underwent the RETIBOOSTER treatment consisted of 33 individuals:
11 individuals with normal skin
11 individuals with oily/combination skin
11 individuals with dry/sensitive skin

### Treatment protocol:

Description of the protocol for the treatment with the preparation of Table 1 :
We conduct a consultation - an interview before the treatment, determining the effect of the treatment, excluding contraindications, we discuss the course of the treatment.
1. Perform make-up removal for the skin of the face, neck and décolleté.
2. Prepare the skin for the treatment with a degreasing preparation
3. Spread the pre-preparation ampoule (Table 6) over the entire surface of the skin of the face, neck and décolleté using a gentle massage technique. Leave it to be absorbed.
4. Protect sensitive and irritation-prone areas (eye corners, nostrils, eyebrow lines, mouth corners) by applying petroleum jelly thereon.
5. Using a brush, evenly distribute the contents of the vial with the preparation according to Table 1 over the entire surface of the face, neck and décolleté. Perform facial massage to excite and stimulate deep tissues, which improves the absorption of active ingredients.
6. Leave the preparation according to Table 1 on the skin for the recommended period of time of 6-10 hours (the appropriate period is determined by a skilled professional, depending on the condition of the skin and its sensitivity (e.g. based on the Fitzpatric scale, Glogau scale and skin type and level of its sensitivity; with sensitive skin, it is recommended to wash the mask off after 6 hours).
7. After a specified period of time, the treated person washes the preparation with lukewarm water at home. Applying any skin care products and make-up on the day of the treatment is not recommended.
8. A control visit is recommended 2-4 days after the treatment.

### Complementary products:

1. Two weeks before the exfoliation treatment begins, it is recommended to use a cream with anti-aging and antioxidant ingredients, e.g. comprising retinol or retinol derivatives, which allows the skin to get used to the application of this ingredient.
2. Two days after washing the mask off (or earlier, if necessary), the treated person soothes inflammation of the skin with a calming cream comprising soothing ingredients.
   The soothing cream is applied 2-3 times a day for 2 weeks (or more often if necessary).
3. 10 days after washing off the mask, it is recommended to start using the preparation, e.g. serum, comprising antioxidant ingredients.
4. For a period of 10-21 days after the treatment, the skin should be protected in the morning and in the afternoon with a preparation comprising UVA and UVB filters, SPF 50+, preferably also comprising ingredients that protect and soothe the skin.

### Results:

The effectiveness of the following therapy has been evaluated:
- antiaging
- brightening discoloration
- reducing seborrhea and shrinking the pores of the skin in the case of oily skins.

As part of the analyzes, the effectiveness of a single treatment was assessed, as well as the effect of the treatment in a serial system. In some volunteers, the difference in the use of the treatment with a preparation according to Table 1 was also assessed in comparison with a similar treatment performed with the use of an emulsion with 4% retinol (without Nicotiana Benthamiana Hexapeptide-40 sh-Polypeptide-76) (used in these individuals earlier).

It should be emphasized that during the study and after its completion, the treatments led to a significant improvement in the condition of the skin in each of the above-mentioned. indications.

In the group of subjects, a visible change was noticeable 10 days after the treatment. The effect was magnified with each subsequent visit. In the study group, the change in brightening effects for skins with discoloration problems was noticeable after the 2nd treatment in the series. It deepened after treatment 3.

In the group of subjects, a visible change in the elimination of problems with excessive seborrhea, acne lesions and shrinkage of skin pores was observed 10 days from the first treatment performed in the series. It deepened after a series of treatments. The analyzed results show that the treatment with the preparation according to Table 1 shows very good effectiveness in the area of anti-aging activities, eliminating the problems of oily skin and brightening superficial and medium-deep discoloration.

In order to assess tolerance to the Retibooster treatment, collecting information on the occurrence of side effects has been planned. It turned out, however, that apart from the inflammation in the form of reddening of the skin, which is expected 7 days after the treatment (which is calmed down by the optional use of an additional soothing preparation), no adverse reaction occurred in the group of subjects.

The degree of satisfaction with the performed treatment was considered both in terms of volunteer and cosmetology specialist. It was based on a 5-point scale, with 5 being the highest grade.

Overall assessment for the treatment:
- in the case of volunteers Retibooster treatment scored 4.5 points, with the skilled professional it scored 4.4 points.

### Summary of the results:

Summarizing the obtained results, it should be noted that both the participants and the skilled professional noticed a significant improvement in effectiveness when using the treatment with the preparation according to Table 1. The group comparing this treatment rated this treatment on a scale of 1-10 (where 1 is a gentle care treatment, and 10 is deeply stimulating and causing significant inflammation), treatment with an emulsion with 4% retinol scored 5 points, and the treatment with the preparation according to Table 1 scored 7 points.

The last element analyzed was the assessment of the influence of the treatment used on the quality of life of the participants of the experiment. The assessment was made on the basis of a comparison of the impact of skin ailments on the quality of life. Assessments were made at the beginning and after 15 weeks. At the beginning, 25% of the participants described their quality of life as normal, after the end of the study, 75% of the subjects noticed that the treatment had a significant impact on their quality of life. During the study, it was observed that the treatment caused exfoliation of the epidermis in a less acute way for the participants of the experiment, lasting several days (i.e. longer than with a typical exfoliating treatment with retinol), but with smaller fragments of flaky epidermis ("snow-sprinkling" effect), compared to the usual expected flaking of the skin.

The study showed the legitimacy of using the treatment with the preparation according to Table 1, in particular in volunteers with an indication for anti-aging care, with oily skin and discoloration.

### Example 7. Apparatus examination of the skin condition after treatment.

The treatment was performed according to the scheme essentially as in Example 6, on a new group of volunteers, in a specialized laboratory conducting research on cosmetic products.

The treatment had the following steps:
1. Make-up removal and degreasing the skin.
2. Applying the composition according to Table 6 and massaging it into the skin - waiting until it is absorbed.
3. Protecting the sensitive areas (eyebrows, eye corners, lips), e.g. by applying petroleum jelly.
4. Measuring 2-3 ml of the preparation according to Table 1 (vial with the solution).
5. Even distribution of the preparation with a brush on the entire surface of the face, neck and décolleté. Leaving the product on the skin (like a mask) for:
   - 8h - face
   - 6h - neck and cleavage
6. After an appropriate exposure time, washing the preparation at home with lukewarm water. No preparations are applied to the skin immediately after washing.

After the treatment, a protective preparation with UV filters, SPF 50+ is used for the day.

### Study groups:

Group 1: normal/combination skin
Group 2: normal/combination skin; additionally, 2 weeks before the treatment, twice a week and 10 days after the treatment, for 2 weeks, twice a week, an anti-wrinkle preparation comprising 0.15 wt.% retinol, 0.15 wt.% retinol palmitate and vitamin C, was used; 10 days after the treatment, for 2 weeks, daily use of an antioxidant preparation comprising ascorbyl glucoside, ethylated vitamin C (up to approx. 15 wt.% of vitamin C) and hyaluronic acid.
Group 3: sensitive/dry skin
Group 4: sensitive/dry skin; additionally, 2 weeks before the treatment, twice a week and 10 days after the treatment, for 2 weeks, twice a week, an anti-wrinkle preparation comprising 0.15 wt. % retinol, 0.15 wt. % retinol palmitate and vitamin C, was used; 10 days after the treatment, for 2 weeks, daily use of an antioxidant preparation comprising ascorbyl glucoside, ethylated vitamin C (up to approx. 15 wt. % of vitamin C) and hyaluronic acid.
Group 5: thick, resistant skins with imperfections, e.g. oily skins
Group 6: thick, resistant skins with imperfections, e.g. oily skins; additionally, 2 weeks before the treatment, twice a week and 10 days after the treatment, for 2 weeks, twice a week, an anti-wrinkle preparation comprising 0.15 wt.% retinol, 0.15 wt.% retinol palmitate and vitamin C was used; just before the treatment, an acid peel was applied for 10 minutes to the entire surface of the face, neck and décolleté, which was then neutralized with a neutralizing preparation; 10 days after the treatment, for 2 weeks, daily use of an antioxidant preparation comprising ascorbyl glucoside, ethylated vitamin C (up to approx. 15 wt. % of vitamin C) and hyaluronic acid

The anti-wrinkle preparation comprises the following ingredients (INCI): Aqua / Water, Cetearyl Ethylhexanoate, Coconut Alkanes, Glycerin, Hydroxyethylacrylate/Sodium Acryloyldimethyl Taurate Copolymer, Polyacrylate, Crosspolymer-6, Polysorbate 20, PEG-35 Castor Oil, Rosa Moschata Seed Oil, Polyisobutene, Water, Propylene Glycol, Helianthus Annuus Seed Oil, Potassium Sorbate, Citric Acid, BHT, Coco-Caprylate/Caprate, Tocopheryl Acetate, Sodium Benzoate, Retinol, Retinyl Palmitate, PEG-7 Trimethylolpropane Coconut Ether, BHA, Bacillus Ferment, Ethyl Ferulate, Polyglyceryl-5 Trioleate, Ethyl Ascorbic Acid, Rosmarinus Officinalis Leaf Extract, Tocopherol, Disodium Uridine Phosphate.

The antioxidant preparation comprises the following ingredients (INCI): Aqua, 3-O-Ethyl Ascorbic Acid, Ascorbyl Glucoside, Squalane, Polyacrylate Crosspolymer-6, Hydroxypinacolone Retinoate, Dimethyl Isosorbide, Iris Florentina Root Extract, Glycerin, Sodium Hyaluronate, Capryl Glycol, Tetrasodium EDTA, Sodium Hydroxide, Sodium Metabisulfite, Butylene Glycol, 1,2-Hexanediol, Phenoxyethanol, Ethylhexylglycerin.

The preparation with UV filters, SPF 50+, used comprises the following ingredients (INCI): Aqua, Diethylamino Hydroxybenzoyl Hexyl Benzoate, Ethylhexyl Methoxycinnamate, Octocrylene, C12-15 Alkyl Benzoate, DiC12-13 Alkyl Malate, Ethylhexyl Triazone, Diisopropyl Adipate, Ethylhexyl Salicylate, Glycerin, Potassium Cetyl Phosphate, Glyceryl Stearate, PEG-100 Stearate, Ectoin, Sorbitan Isostearate, Polygonum Aviculare Extract,Tocopheryl Acetate, Titanium Dioxide, Xanthan Gum, Quartz, Magnesium Aluminum Silicate, Tetrasodium EDTA, Phenoxyethanol, Ethylhexylglycerin, Potassium Sorbate, Sodium Benzoate.

If necessary, after the treatment, the volunteers could use, as often as necessary, a soothing preparation comprising the following ingredients: (INCI): Aqua, Gluconolactone, C15-19 Alkane, Lactobionic Acid, Propylene Glycol, Glycerin, Cetearyl Alcohol, Glyceryl Stearate, PEG-100 Stearate, Ectoin, C12-15 Alkyl Benzoate, Ethylhexyl Stearate, Dimethicone, Macadamia Ternifolia Seed Oil, Palmitic Acid, Stearic Acid, Paraffin, Petrolatum, Arginine, Octyldodecanol, Steareth-2, Laminaria Hyperborea Extract, Sodium Hyaluronate, Tocopherol, Caprylic/Capric Triglyceride, Olus Oil, Myristic Acid, Lauric Acid, Polyacrylamide, Oleic Acid, C13-14 Isoparaffin, Dimethicone Crosspolymer, Laureth-7, Caprylyl Glycol, Butylene Glycol, Tetrasodium EDTA, 1,2- Hexanediol, Phenoxyethanol, Ethylhexylglycerin.

The volunteers underwent a series of 3 treatments at monthly intervals.

The volunteers assessed the perceived effectiveness of the treatment and its properties, based on parameters that they could observe or feel.

The apparatus studies were carried out:
o in 20 individuals divided into 6 groups;
o in standardized environmental conditions, i.e. temperature 20 - 25 °C and relative humidity 40 - 60%;
o using specialized measuring equipment from Courage & Khazaka;
   - the measurement of skin surface and wrinkles smoothing was performed with a device called Visioscan ^{®} VC20plus;
   - measurement of elasticity and firmness was performed with a device called Cutometer^{®} DUAL MPA580;
   - measurement of the number and size of the excessively dilated orifices of the sebaceous glands "pores" was performed with a device called Visioface^{®} RD;
   - the discoloration measurement was performed with a device called Mexameter ^{®} MX18;
   - photos showing the improvement of the appearance of the facial skin, i.e. brightening of discoloration and smoothing out wrinkles, were taken with a device called Visioface ^{®} RD;
Apparatus studies were performed before the start of the series of treatments and after the first treatment, during exfoliation and before and after the third treatment;
Measurements were carried out on a selected area of the facial skin - the same before and after the first treatment, during exfoliation and before and after the third treatment;
Measurements were carried out on the skin of the face properly prepared for the tests, i.e. clean skin, not treated with any cosmetics (initial measurement and other measurements).
Application study was aimed at subjective assessment of application properties. Apparatus studies were aimed at objectively confirming the declared operation of the treatment and determining the parameters illustrating the condition of the skin, i.e.: the level of smoothing, the level of elasticity, the size of wrinkles and discoloration, and the measurement of the number and size of excessively dilated sebaceous glands "pores".

The inclusion and exclusion criteria are shown below in Table 7:

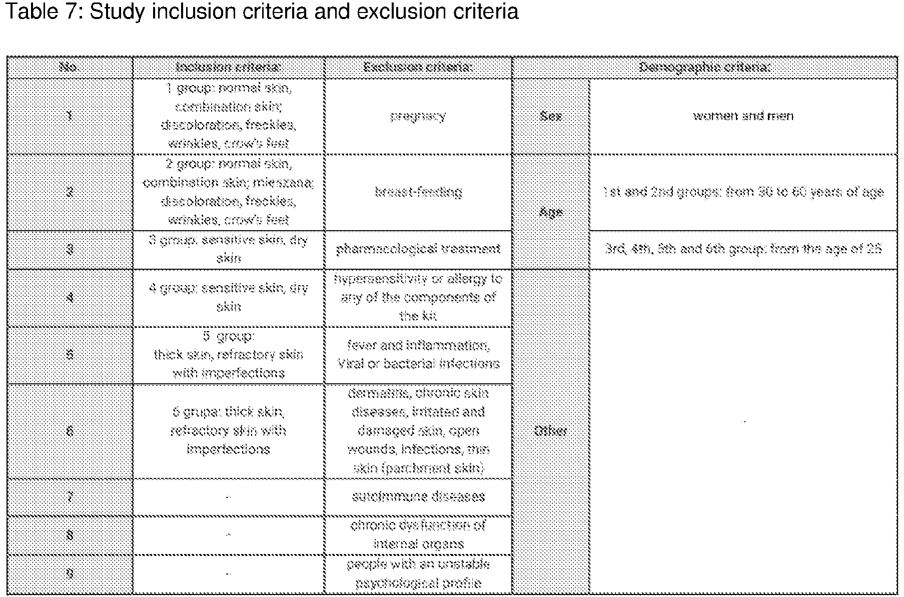

### Results

Volunteers from groups 1, 3 and 5 after the first treatment assessed that the treatment had an effect:
- brightening the skin (definitely yes 40% of the respondents, rather yes 50% of the respondents);
- tightening the skin (definitely yes, 80% of respondents);
- smoothing the skin (definitely yes, 70% of respondents);
- firming the skin (definitely yes 30% of the respondents, rather yes 40% of the respondents);
- regenerating the skin (definitely yes 40% of the respondents, rather yes 30% of the respondents);
- improving the overall appearance and condition of the skin (definitely yes 50% of the respondents, rather yes 50% of the respondents).

Volunteers from groups 2, 4 and 6 after the first treatment assessed that the treatment had an effect:
- brightening the skin (definitely yes 40% of the respondents, rather yes 40% of the respondents);
- tightening the skin (definitely yes 40% of the respondents, rather yes 40% of the respondents);
- smoothing the skin (definitely yes 60% of the respondents, rather yes 30% of the respondents);
- tightening the skin (definitely yes 50% of the respondents, rather yes 10% of the respondents);
- regenerating the skin (definitely yes 30% of the respondents, rather yes 60% of the respondents);
- improving the overall appearance and condition of the skin (definitely yes 50% of the respondents, rather yes 50% of the respondents).

The volunteers also assessed the degree of epidermis exfoliation. The first treatment scores are summarized in Table 8 below.

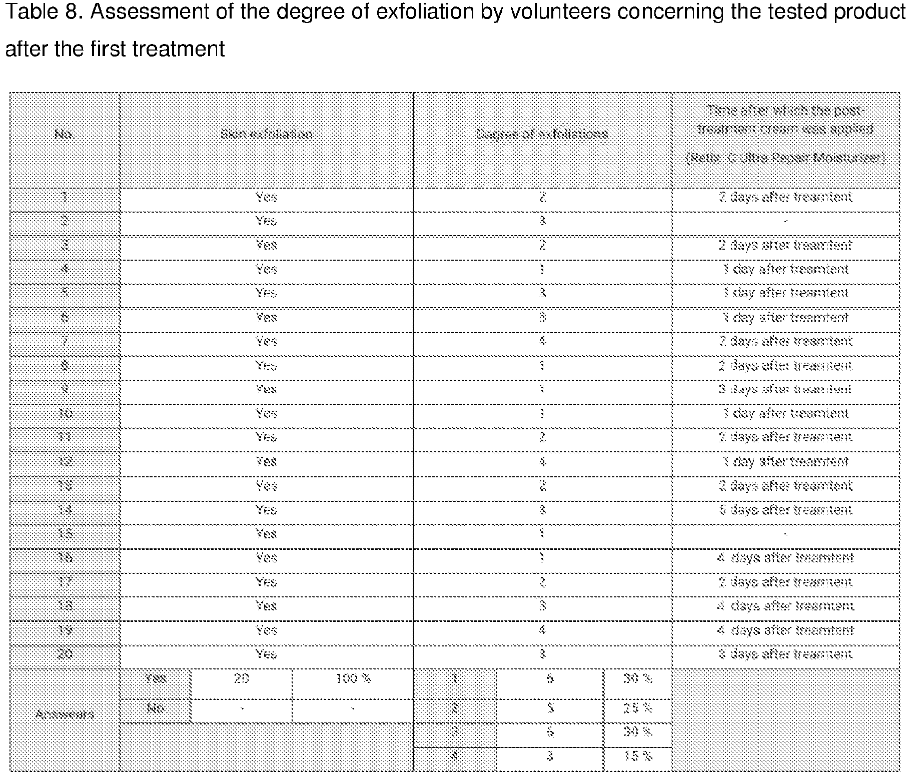

Assessment of the degree of exfoliation:
1- minor exfoliation around the mouth and nose
2- exfoliation all over the face, resembling "coconut flakes"
3- fine-flake exfoliation all over the face, with flakes less than 50mm in diameter
4- exfoliation all over the face, with flakes over 50 mm to 100 mm in diameter.

Exemplary photos of the skin condition and exfoliation after the first treatment are shown in Fig. 1.

None of the volunteers reported any adverse effects unrelated to the intended effect of the procedure (exfoliation of the epidermis, some degree of redness or burning of the skin).

### Results of apparatus studies after first treatment

Description of symbols in the Tables:
P0 - initial measurement before starting the application of the preparation
P1 - measurement after the first treatment
BP - no improvement, insignificant difference resulting from device measurement error

The results for each group are presented in the tables below:

### Group 1

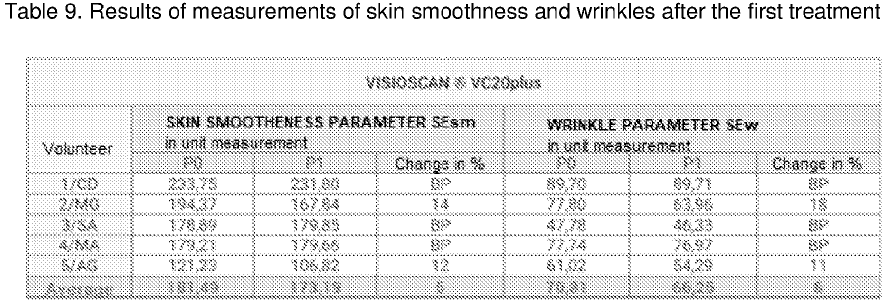

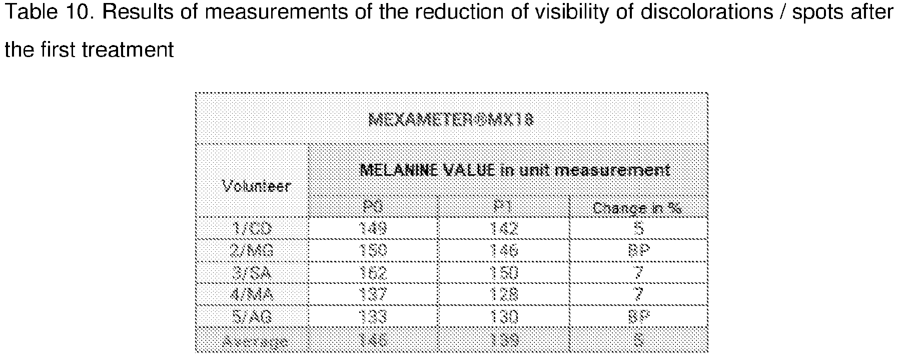

### CONCLUSIONS resulting from apparatus studies

Already after the first treatment, positive changes in the appearance of the skin of the volunteers are noticeable. The surface of the skin has been slightly smoothed, making unevenness and wrinkles less visible. The skin tone has been evened out thanks to the brightening of discoloration. This effect was observed in 2 out of 5 individuals in the group, in the remaining individuals the changes were not noticeable.

### Group 2

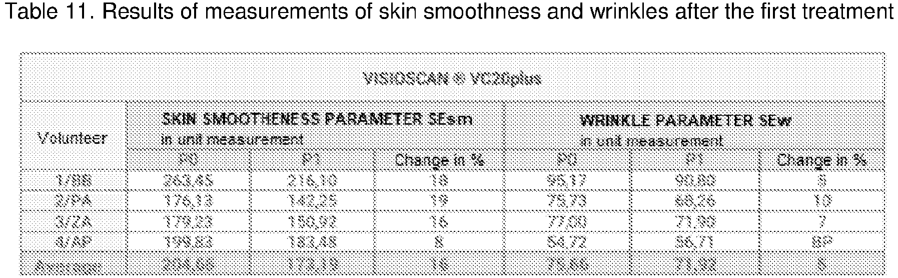

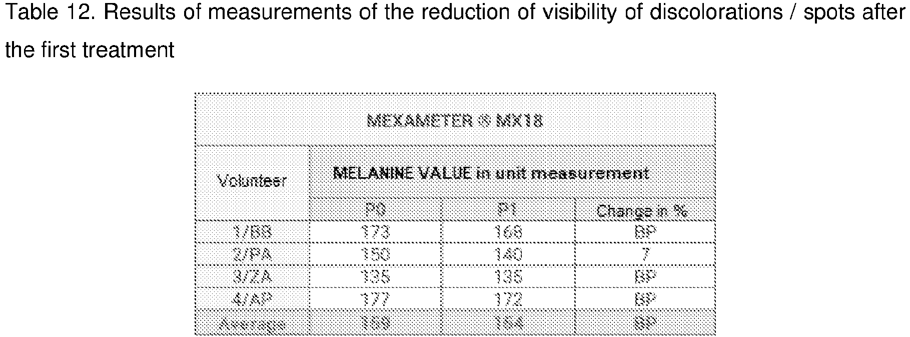

### CONCLUSIONS resulting from apparatus studies

Already after the first treatment, positive changes in the appearance of the skin of the volunteers are noticeable. The surface of the skin has smoothed out, making unevenness and wrinkles less visible. On the other hand, there were no clear changes in the alignment of the skin tone - improvement only in 1 individual.

### Group 3

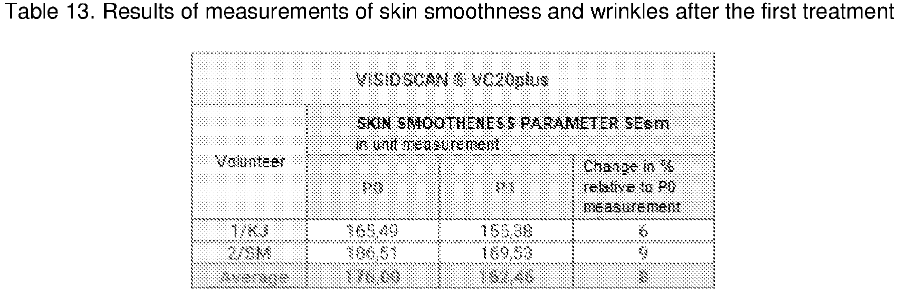

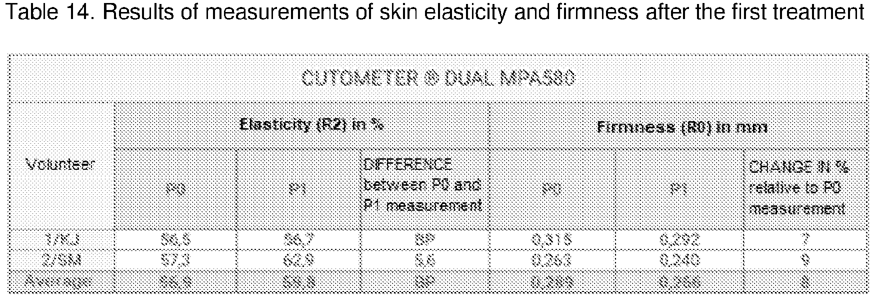

### CONCLUSIONS resulting from apparatus studies

Already after the first treatment, positive changes in the appearance of the skin of the volunteers are noticeable. The skin surface was smoothed and its firmness increased in both subjects.

### Group 4

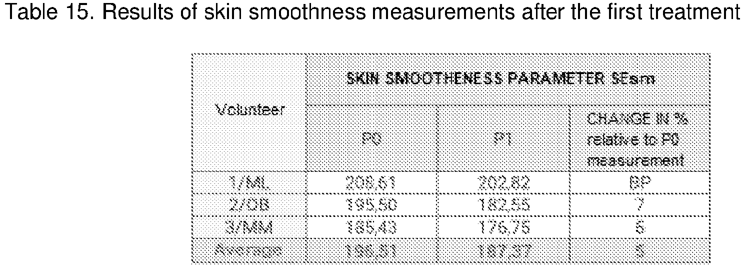

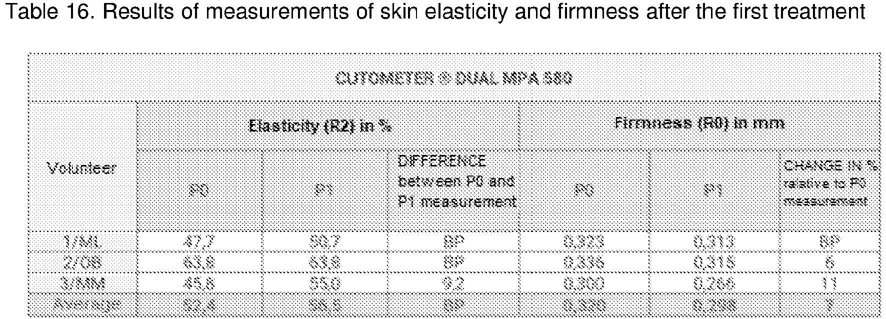

### CONCLUSIONS resulting from apparatus studies

Already after the first treatment, positive changes in the appearance of the skin of the volunteers are noticeable. The surface of the skin was smoothed in 2 individuals, and its firmness also increased in two of the subjects.

### Group 5

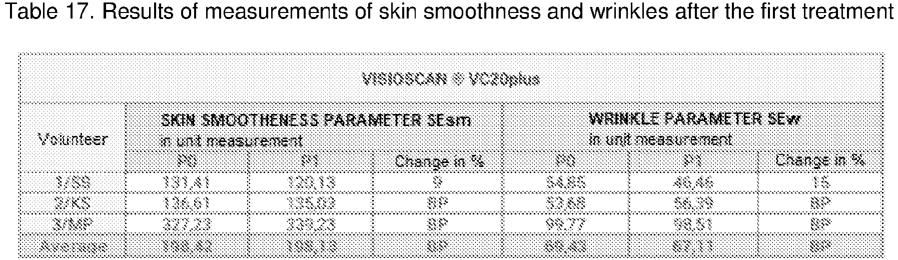

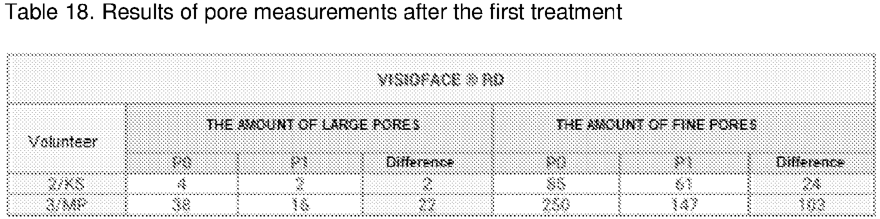

### CONCLUSIONS resulting from apparatus studies

In the studied group of individuals with problematic skin, with imperfections, eczema, excessively dilated orifices of the sebaceous glands, a single treatment did not clearly and measurably contribute to increasing the smoothness of the skin or reducing the visibility of wrinkles. Positive changes were observed only in the case of "pores", which in 2 out of 3 individuals clearly decreased.

### Group 6

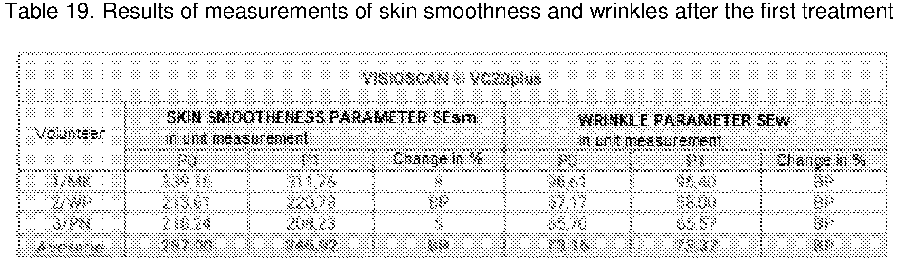

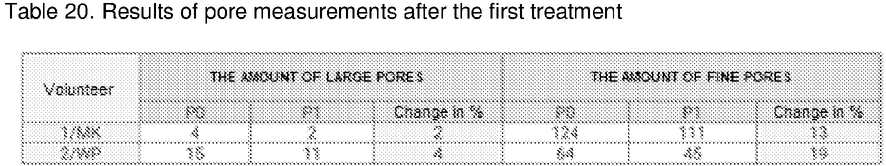

### CONCLUSIONS resulting from apparatus studies

In the studied group of individuals with problematic skin, with imperfections, eczema, excessively dilated orifices of the sebaceous glands, a single treatment did not clearly and measurably contribute to increasing the smoothness of the skin or reducing the visibility of wrinkles. Positive changes were observed only in the case of "pores", which in 2 out of 3 individuals clearly decreased. Skin smoothness improved measurably in 2 of 3 individuals, but on average it did not change in the group.

### Application studies after 3 months:

**After a series of 3 treatments, the volunteers found that the treatment brings the following results:**
- increased skin tension, confirmed by 85.7% of the volunteers
- skin smoothing confirmed by 95.2% of the volunteers
- skin firming confirmed by 81% of the volunteers
- skin refreshment confirmed by 100% of volunteers
- reduction of signs of aging confirmed by 81% of volunteers
- visible reduction of wrinkles and furrows, confirmed by 81% of the volunteers
- skin regeneration confirmed by 95.2% of volunteers
- lifting effect confirmed by 81% of the volunteers
- improvement of the overall appearance and condition of the skin, confirmed by 100% of the volunteers
- increased skin elasticity, confirmed by 76.2% of the volunteers
- reduction of imperfections confirmed by 76.2% of the volunteers
- reduction of lesions after imperfections (acne), confirmed by 81% of the volunteers
- even skin tone confirmed by 85.7% of the volunteers
- reduction of discoloration confirmed by 71.4% of the volunteers
- improvement of the skin structure, confirmed by 71.4% of the volunteers

Futhermore,
- 76.2% of volunteers would perform the treatment again
- 76.2% of the volunteers assessed the treatment positively
- 85.7% of the volunteers would recommend the treatment to their friends

According to the cosmetologist, the treatment is simple to perform, and it is recommended for mature, acne-prone and sensitive skin.

The volunteers also assessed the degree of epidermis exfoliation. Ratings after a series of 3 treatments are summarized in Table 21 below.

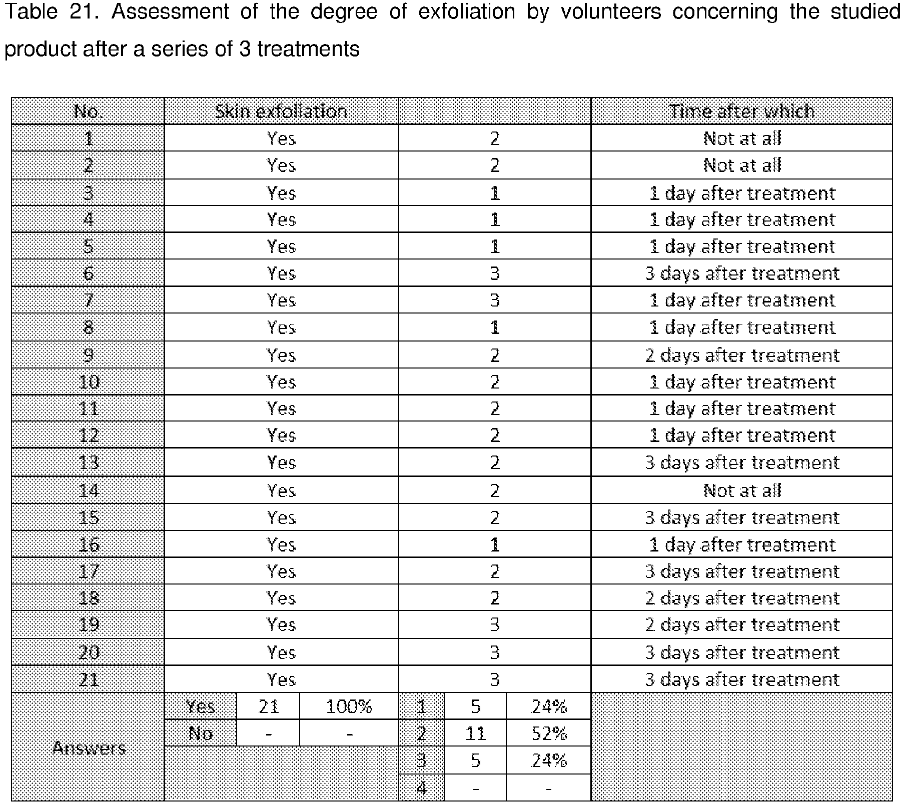

Assessment of the degree of exfoliation:
1- minor exfoliation around the mouth and nose
2- exfoliation all over the face, resembling "coconut flakes"
3- fine-flake exfoliation all over the face, with flakes less than 50mm in diameter
4- exfoliation all over the face, with flakes over 50 mm to 100 mm in diameter
21 individuals out of 21 volunteers participating in the study did not report any unforeseen effects not related to the proper course of the treatment.

The results of the apparatus tests after the first and third treatment

Description of symbols in the Tables:
P0 - initial measurement before starting the application of the preparation
P1 - measurement after the first treatment
P2 - measurement after the third treatment
BP - no improvement, insignificant difference resulting from device measurement error

**The results of apparatus studies for Groups I and II after the first and third treatment**

**The results for the group with normal skin, combination skin, skin with discoloration, freckles and wrinkles.**

### Skin smoothness parameter

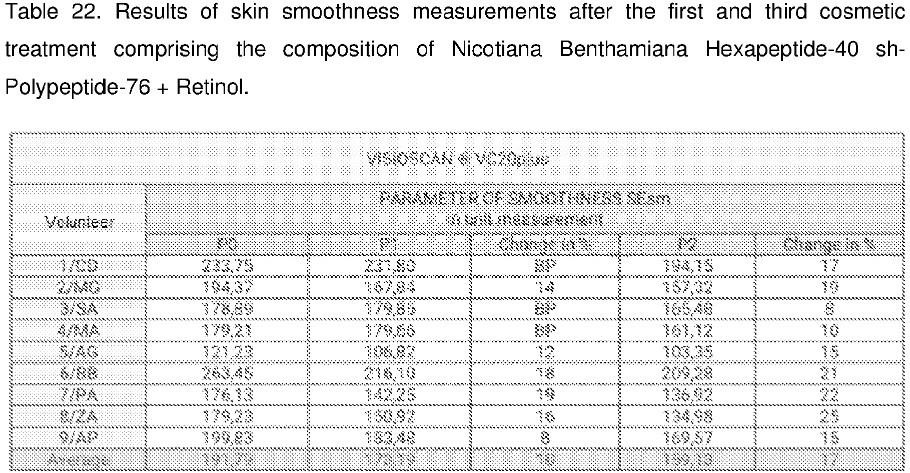

### A DECREASE in the SEsm PARAMETER value indicates an improvement - increase in the smoothness of the skin

Changes below 5% are treated as due to measurement error and marked with BP (no improvement).

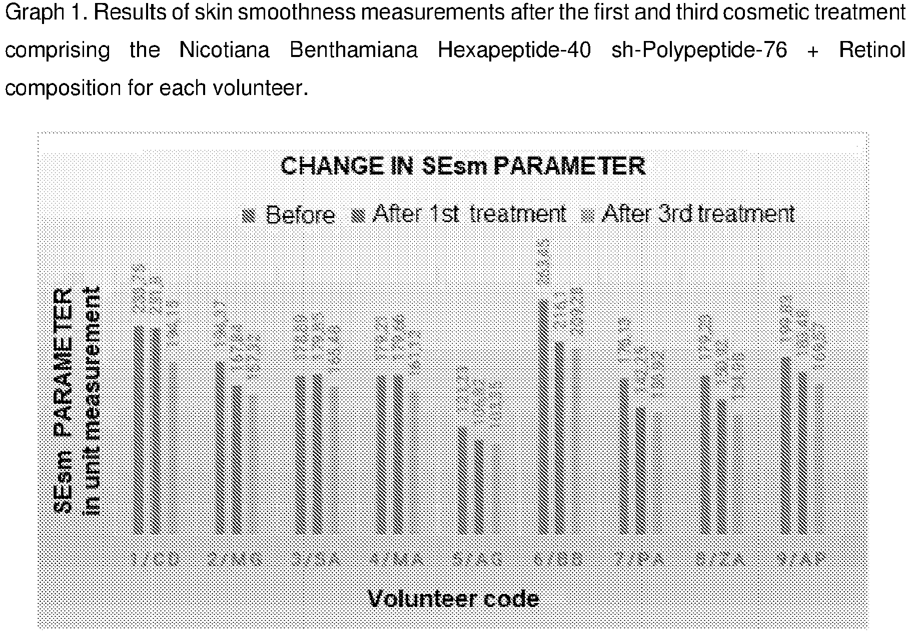

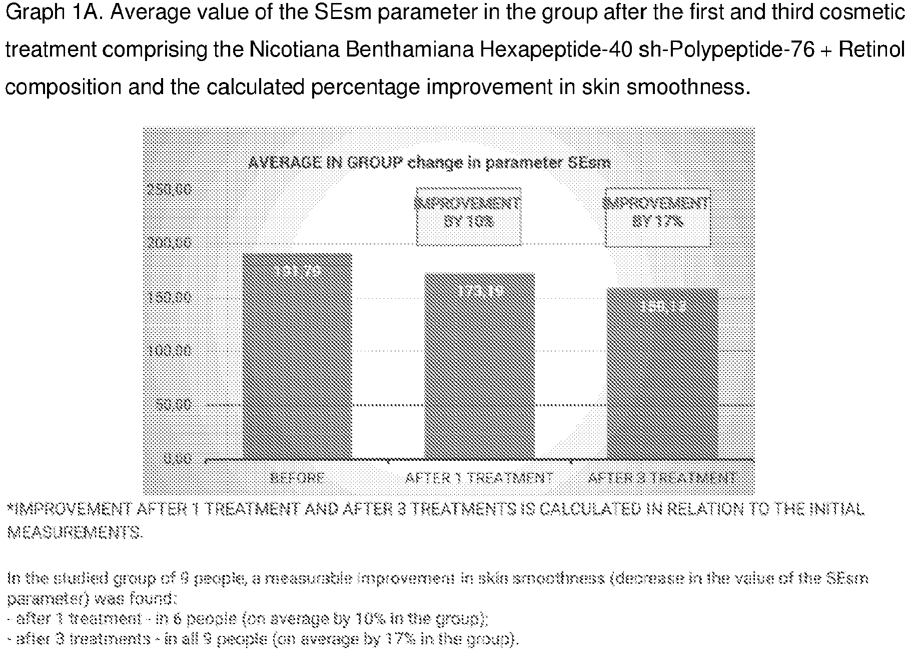

### WRINKLE MEASUREMENT

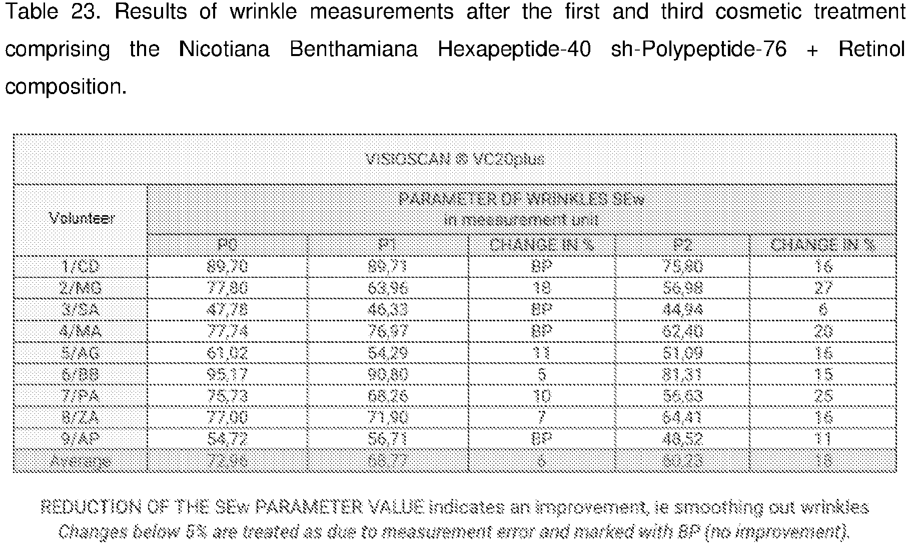

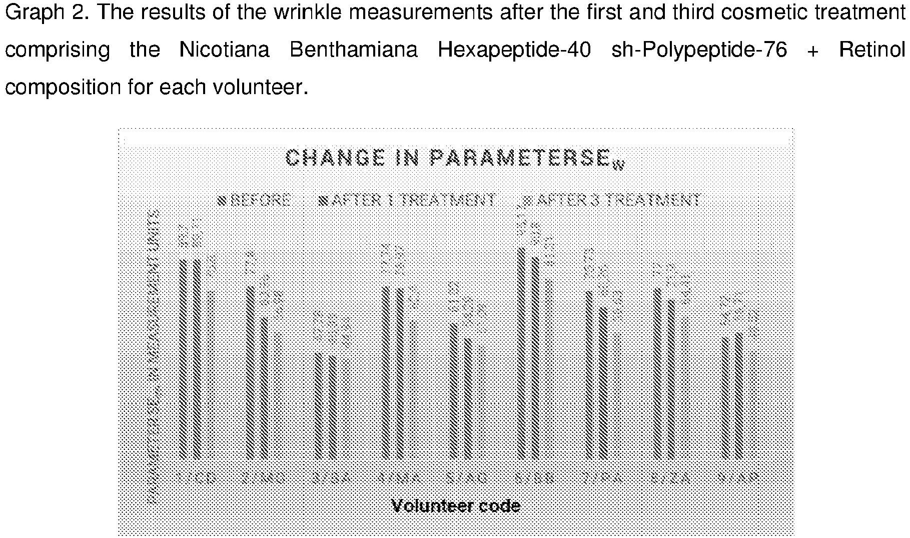

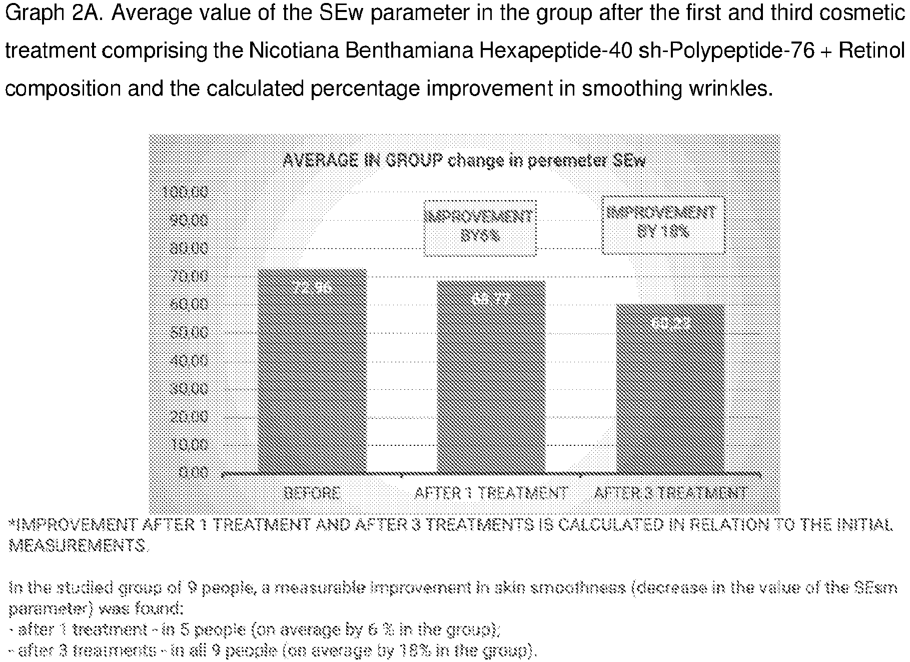

### Discoloration measurement

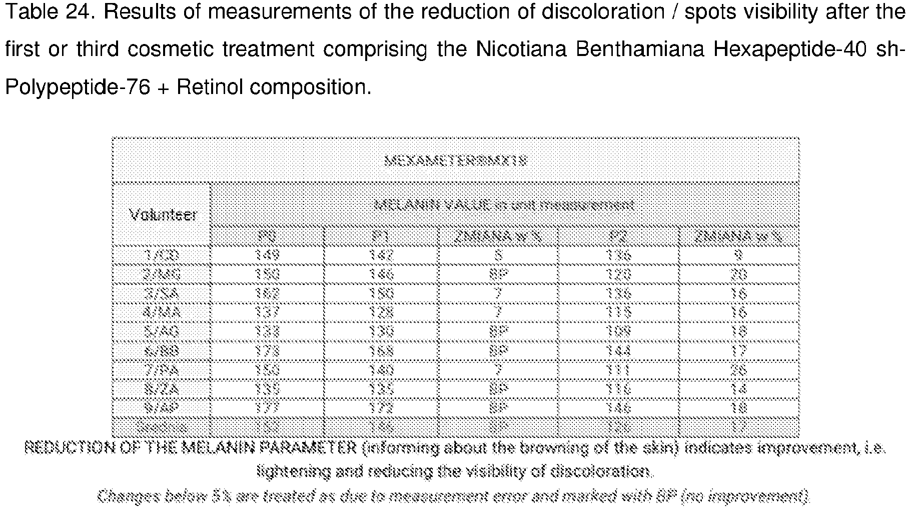

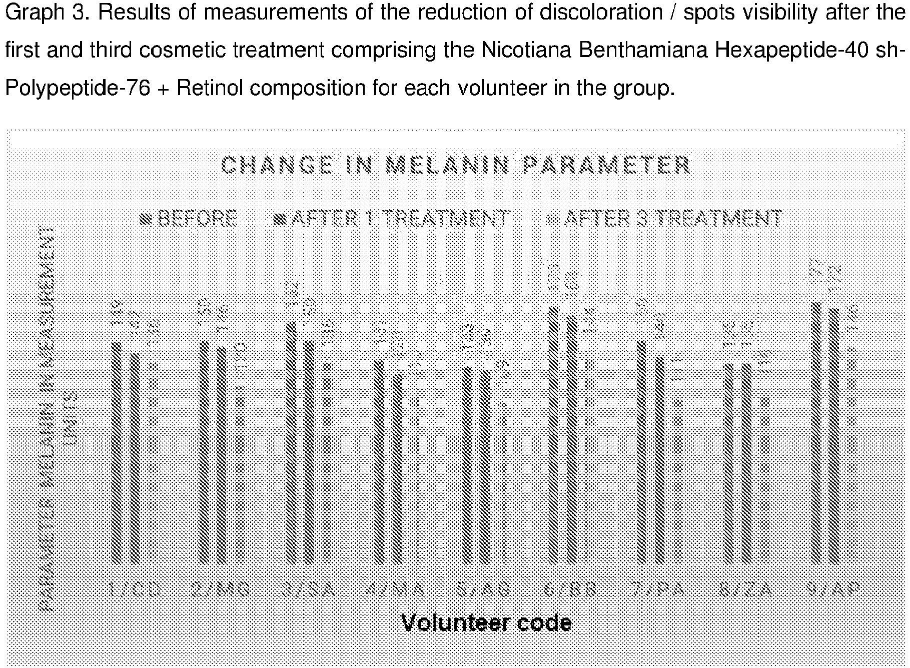

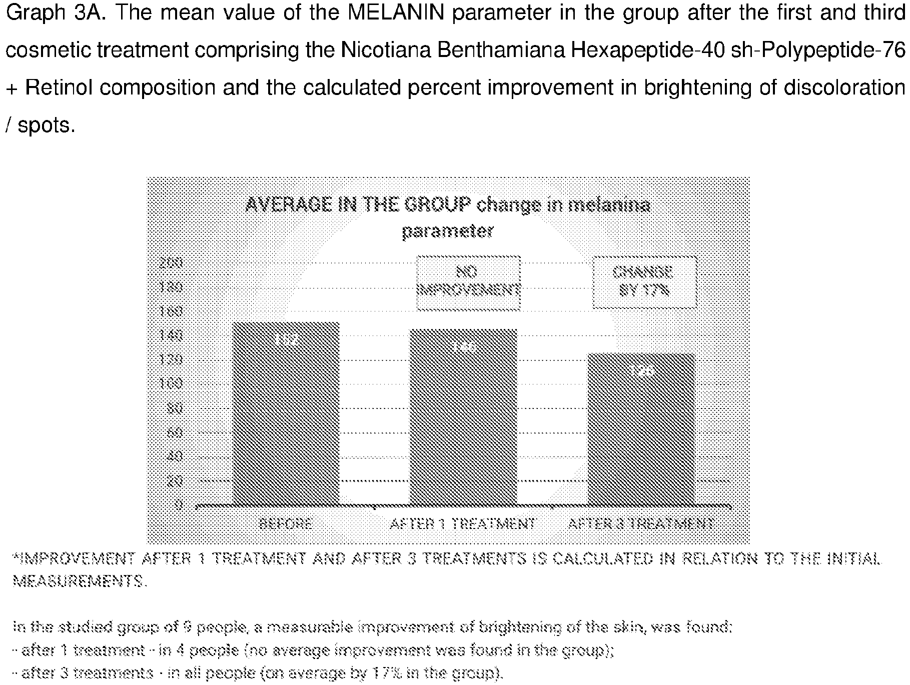

### Conclusions resulting from application studies:

Already after the first treatment, positive changes in the appearance of the skin of the volunteers are noticeable, i.e.
- Gentle smoothing of the skin surface - on average by 10% in the group.
- Less visible unevenness of the skin surface, including wrinkles - on average by 6% in the group.

*NOTE: the above changes were observed in 6 individuals in the group (changes in the SEsm parameter) and 5 individuals in the group (changes in the SEw parameter), in the remaining individuals the changes were not noticeable.*

The continuation of the treatments contributed to the improvement of the appearance and condition of the facial skin. For all 9 individuals in the group:
- The surface of the skin is smoothed - by an average of 17%.
- Wrinkles became less visible, smoothed - by an average of 18%.
- The skin tone has been evened out thanks to the brightening of discolorations / spots - on average by 17%.

**The results of apparatus studies for Groups III and IV after the first and third treatment**

### Results for the group with dry and sensitive skin.

### Measurement of skin elasticity

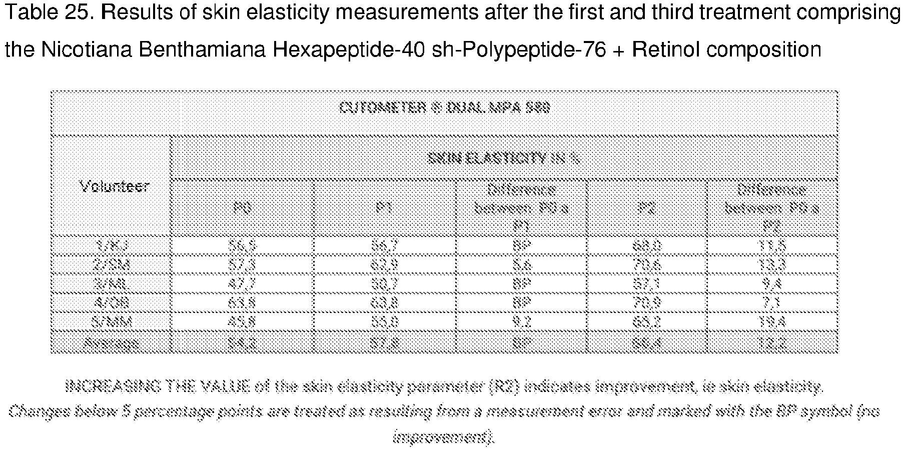

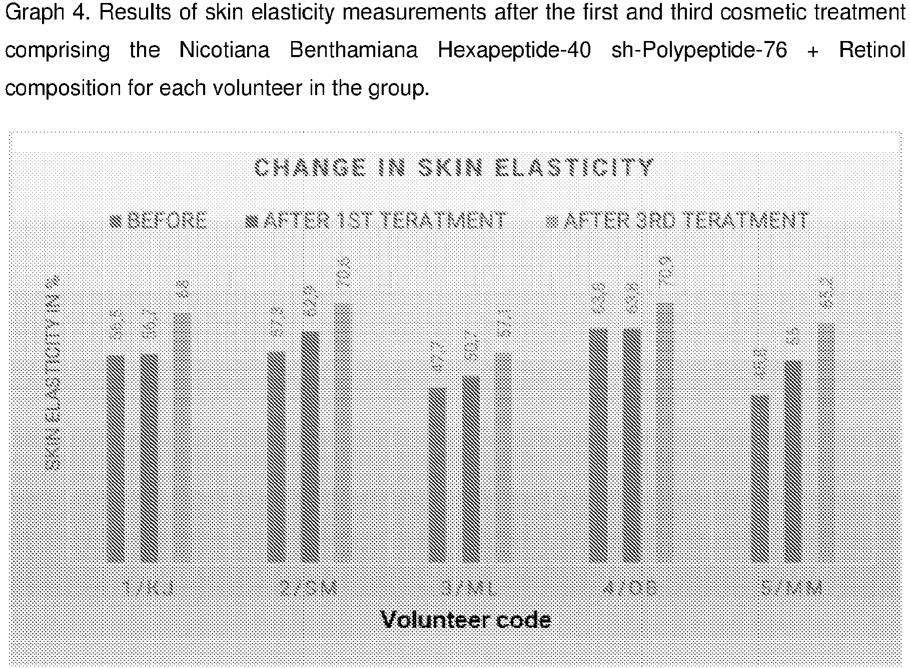

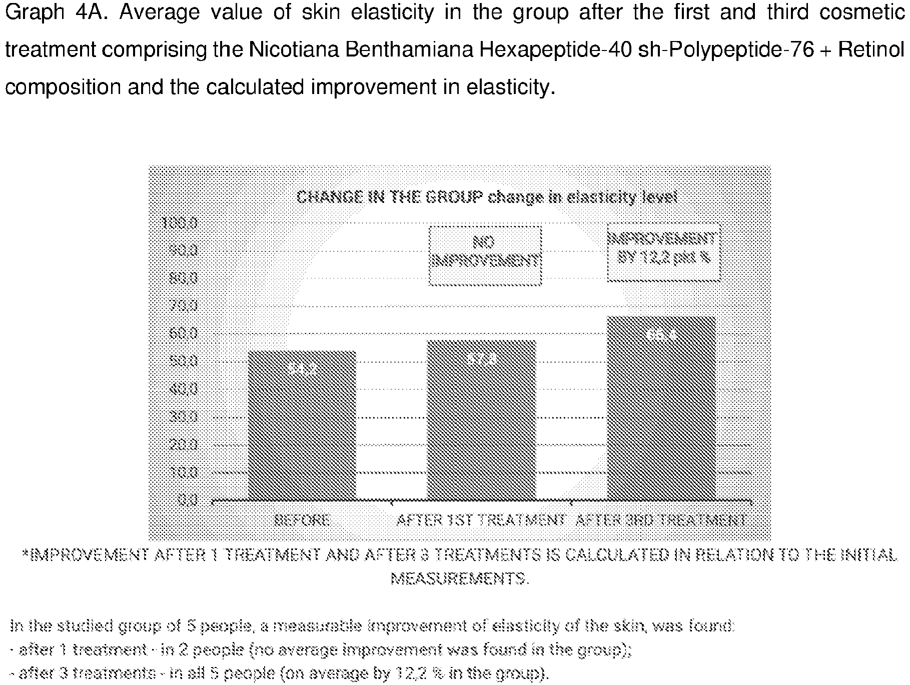

### Skin firmness measurement

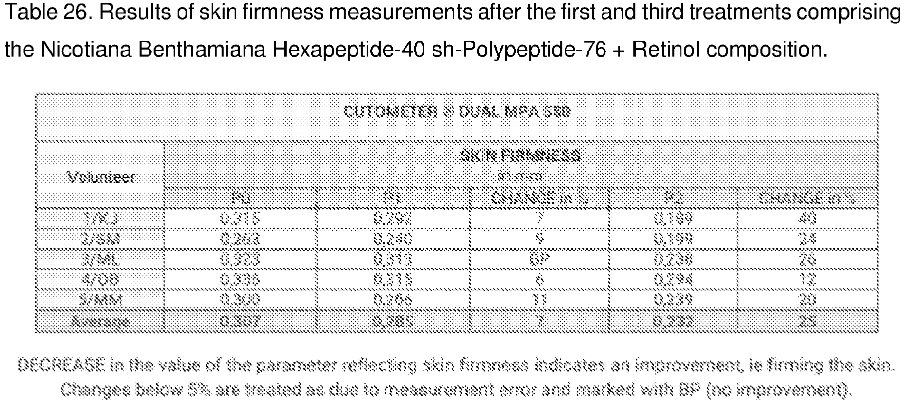

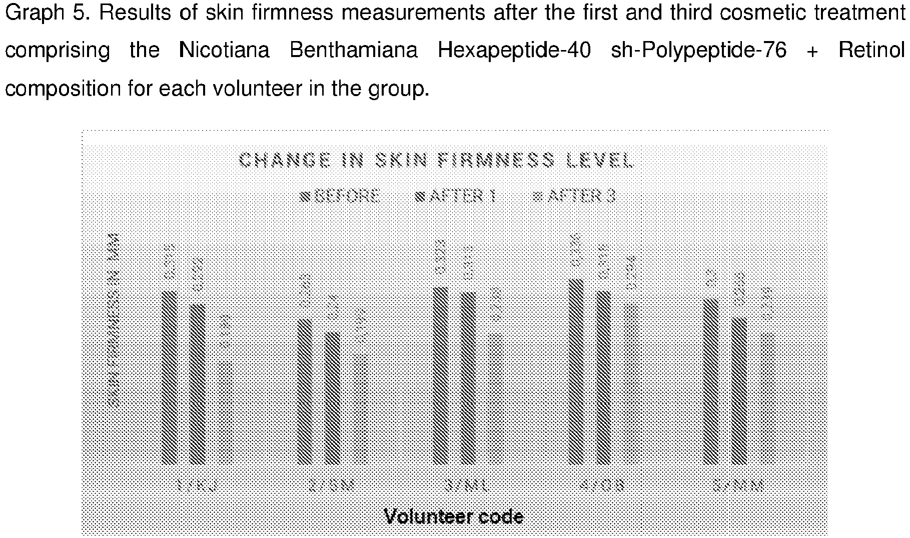

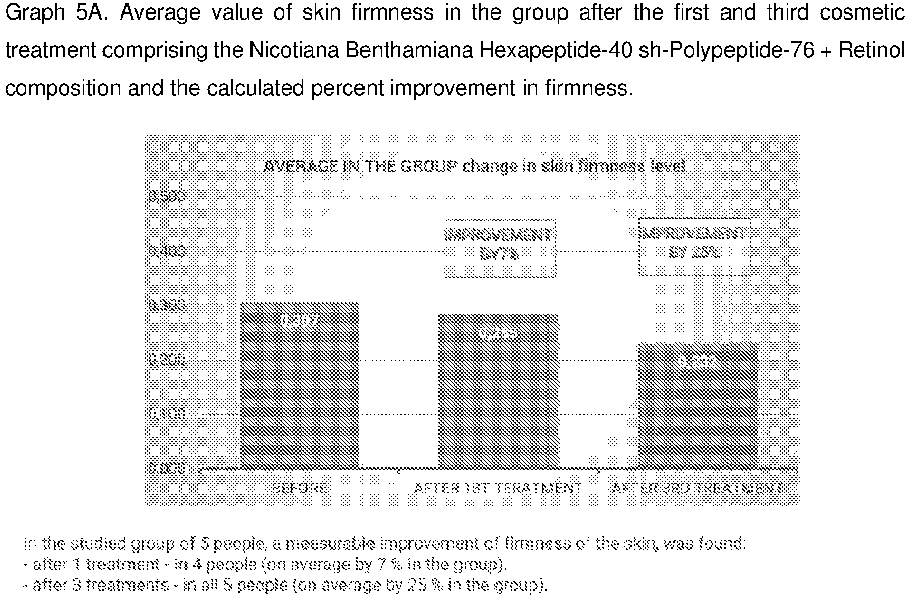

### Measurement of the skin smoothness

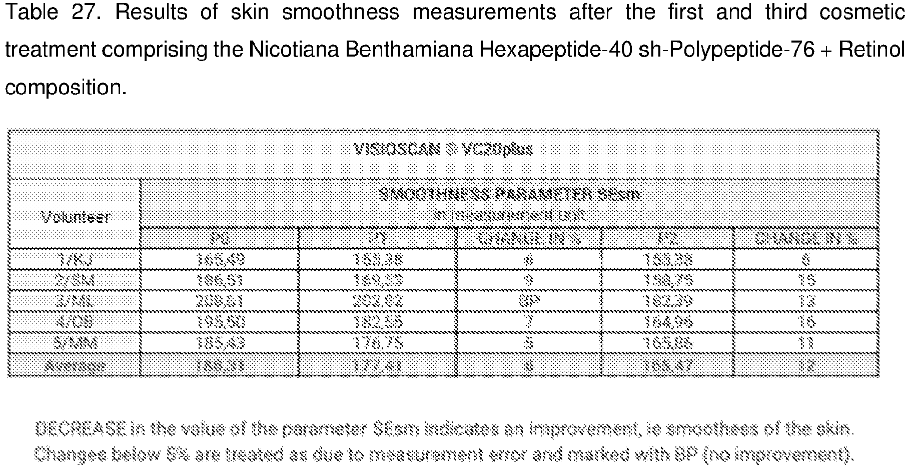

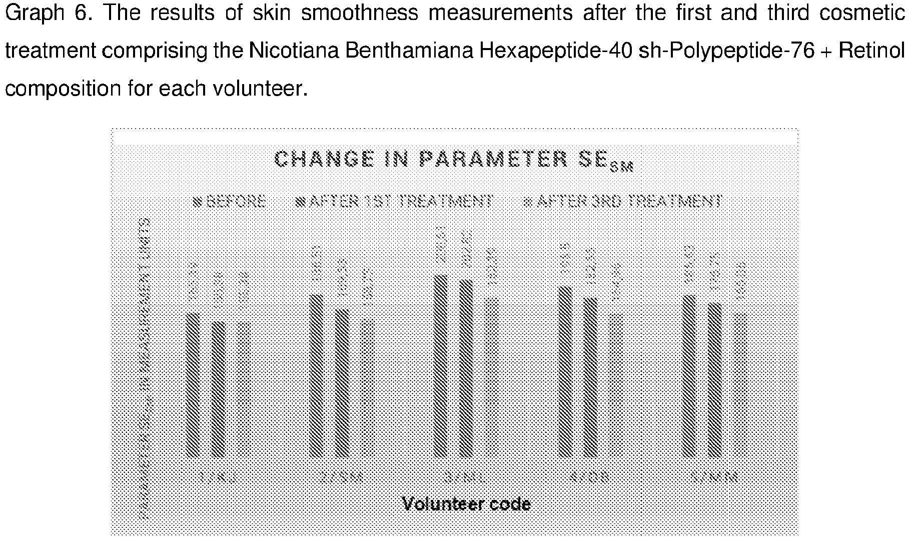

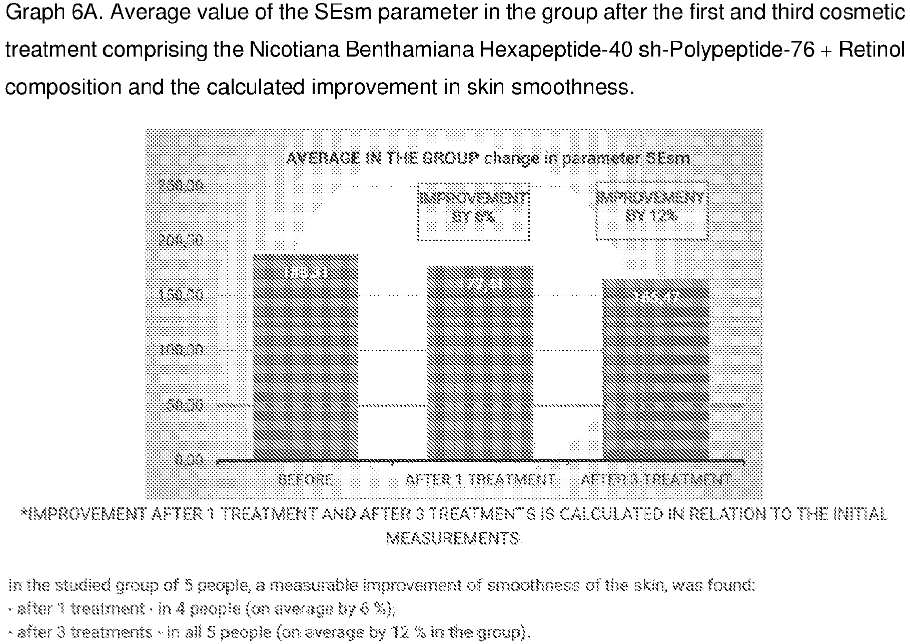

### Conclusions resulting from the apparatus studies:

Already after the first treatment, positive changes in the appearance of the skin of the volunteers are noticeable, i.e.
- Smoothing the skin surface - on average by 6%.
- The skin is firmer - on average by 7%.

The continuation of the treatments contributed to the improvement of the appearance and condition of the facial skin. For all 5 individuals in the group:
- The skin surface has been smoothed more clearly - on average by 12%.
- The skin has become even firmer - on average by 25%.
- The skin became more elastic - on average by 12.2 percentage points.

**The results of apparatus studies for Groups V and VI after the first and third treatment**

### Results for thick, resistant skins with imperfections.

### Measurement of the skin smoothness

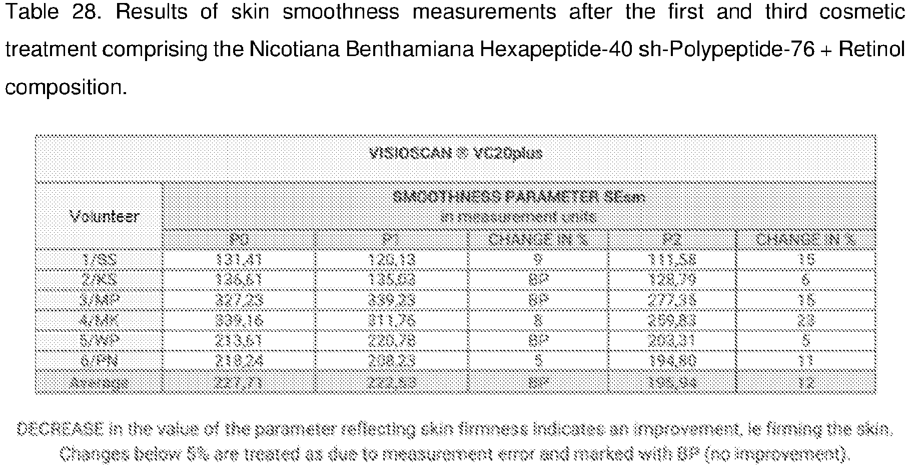

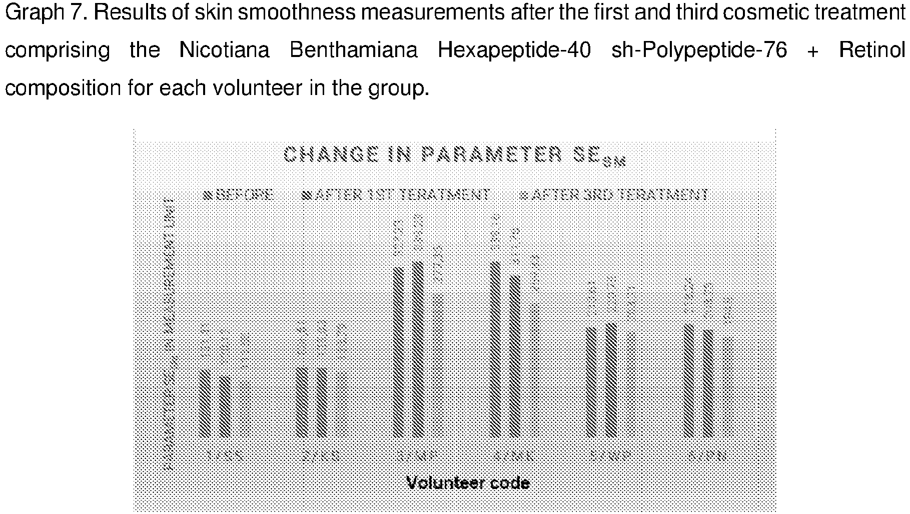

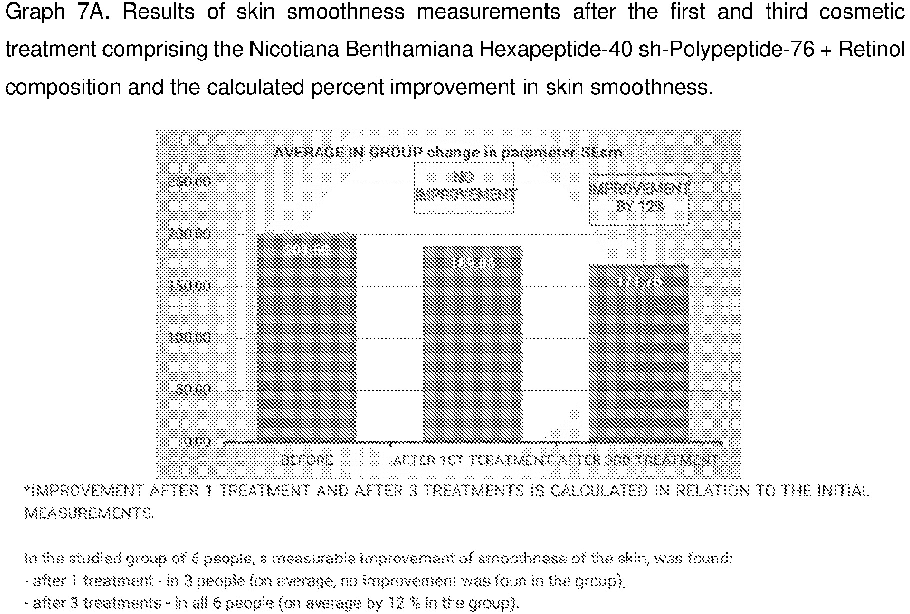

### Wrinkle measurement

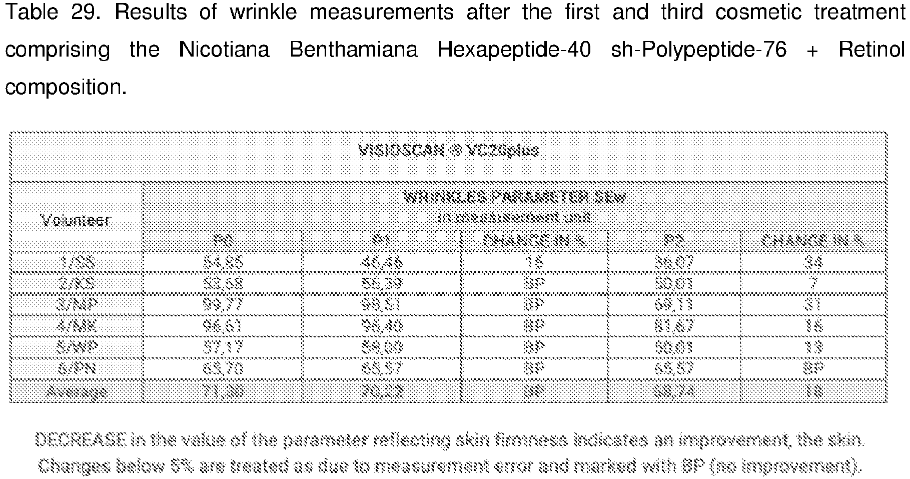

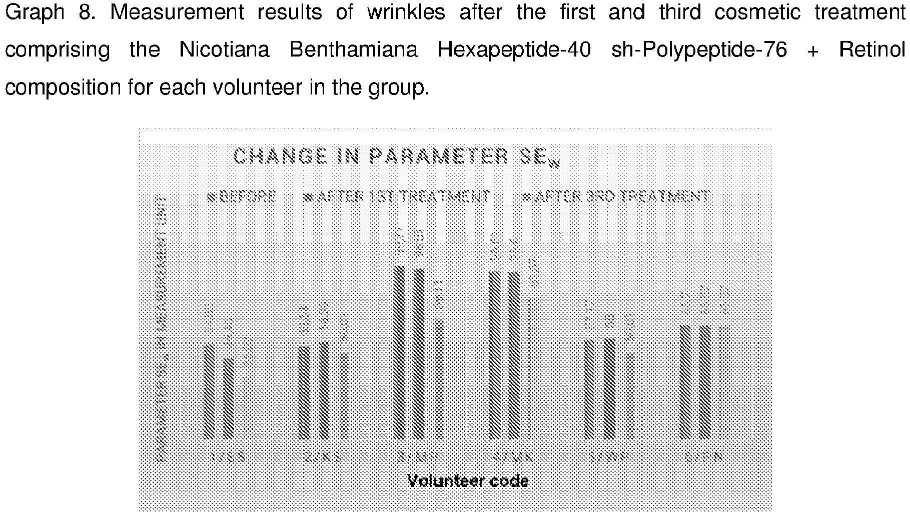

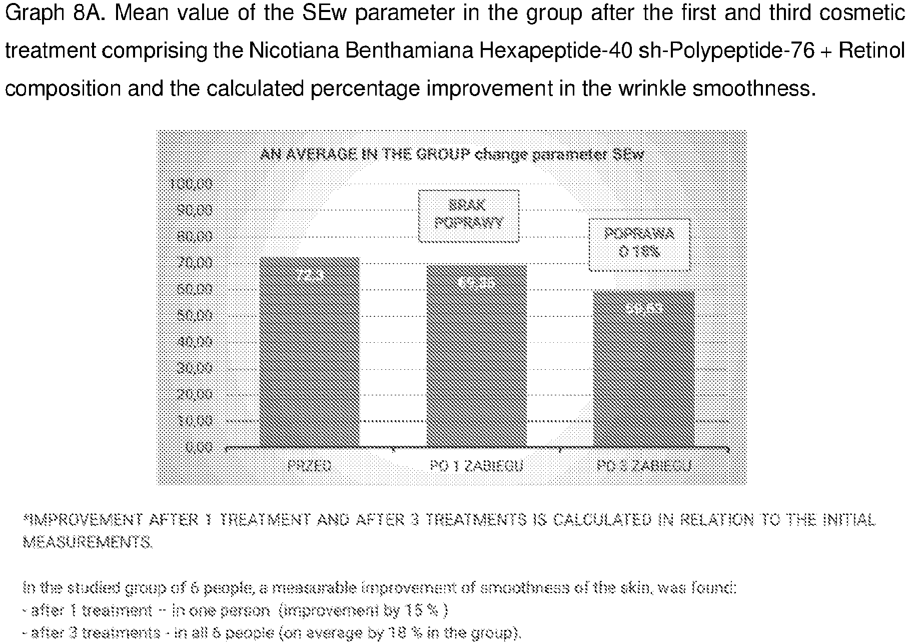

### Total amount of pores

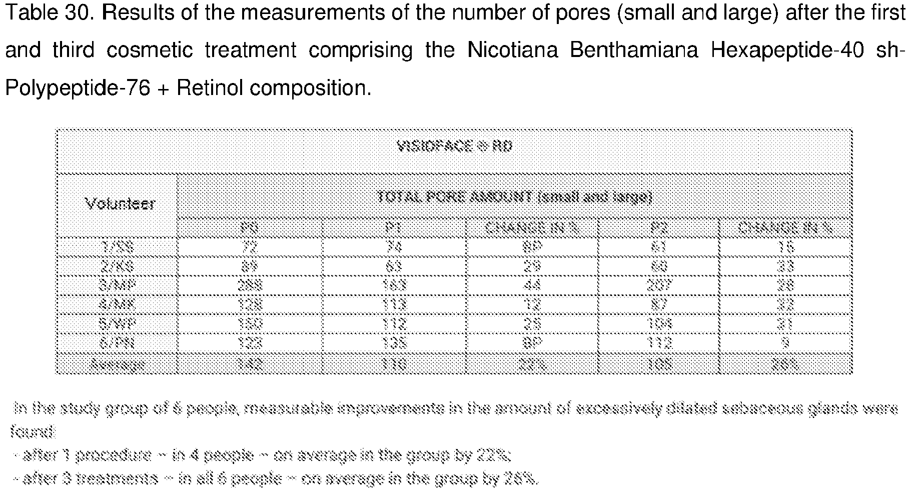

### Conclusions resulting from the apparatus studies:

A single treatment did not contribute to any significant changes in the skin smoothness of the volunteers. On the other hand, positive changes were observed in the case of "pores", the number of which decreased significantly in 4 out of 6 subjects.

The continuation of the treatments contributed to the improvement of the appearance and condition of the facial skin. For all 5 individuals in the group:
- The surface of the skin is smoothed - on average by 12%.
- Wrinkles have become noticeably less visible, smoother - on average by 18%.
- The excessively dilated openings of the sebaceous glands narrowed and became less visible.
- The overall appearance of the blemished complexion has improved.

In the opinion of the cosmetologist, the product can be used by individuals with mature, sensitive skin, as well as with acne problems. In the dermatologist's opinion, the product can be used for all skin types, including sensitive ones.

This is an unexpected effect because retinol products are not recommended for all skin types, especially not for sensitive skins. Our research also confirms the positive effect on acne.

## Claims

1. A composition having a hydroalcoholic base, comprising retinol, **wherein** the composition comprises retinol in a concentration of 3 - 7 wt.%, and an ingredient having INCI name of Nicotiana Benthamiana Hexapeptide-40 sh-Polypeptide-76.

2. The composition according to claim 1, **wherein** the composition comprises retinol in a concentration of 3.5 - 5 wt.%.

3. The composition according to claim 1 or 2, **wherein** the composition comprises the Nicotiana Benthamiana Hexapeptide-40 sh-Polypeptide-76 ingredient in the amount of 0.000002 - 0.00003 wt.%.

4. A method of non-therapeutic exfoliation of the outer layers of the epidermis, **wherein** the method comprises the steps of:
a) optionally cleansing or removing make-up from the skin;
b) optionally degreasing the skin, optionally also performing a chemical or mechanical peeling;
c) applying a preparation comprising antioxidant ingredients;
d) protecting sensitive areas such as the corners of the eyes, the wings of the nose, the lines of the eyebrows, the corners of the mouth;
e) applying the composition as defined in any one of claims 1-3;
wherein the composition applied in step e) is not washed from the skin for a period of at least 6-10 hours.

5. The method of non-therapeutic exfoliation of the outer layers of the epidermis according to claim 4, **wherein** antioxidant ingredients of preparation applied in step c) are vitamin C and ferulic acid.

6. The method of non-therapeutic exfoliation of the outer layers of the epidermis according to claim 4 or 5, **wherein** the method is performed by a skilled professional.

7. The method of non-therapeutic exfoliation of the outer layers of the epidermis according to any one of claims 4-6, **wherein** the composition applied in step e) is not washed from the skin for a period of 6-10 hours.

8. The method of non-therapeutic exfoliation of the outer layers of the epidermis according to any one of claims 4-7, **wherein** the method comprises the step of a) cleansing or removing make-up from the skin and/or the step of b) degreasing the skin, optionally also performing a chemical or mechanical peeling.

## Patentansprüche

1. Eine Zusammensetzung auf hydroalkoholischer Basis, umfassend Retinol, **wobei** die Zusammensetzung Retinol in einer Konzentration von 3-7 Gew.-% und einen Inhaltsstoff mit der INCI-Bezeichnung Nicotiana Benthamiana Hexapeptide-40 sh-Polypeptide-76 umfasst.

2. Zusammensetzung nach Anspruch 1, **wobei** die Zusammensetzung Retinol in einer Konzentration von 3,5-5 Gew.-% umfasst.

3. Zusammensetzung nach Anspruch 1 oder 2, **wobei** die Zusammensetzung den Inhaltsstoff Nicotiana Benthamiana Hexapeptide-40 sh-Polypeptide-76 in einer Menge von 0,000002-0,00003 Gew.-% umfasst.

4. Ein Verfahren zur nicht-therapeutischen Exfoliation der äußeren Schichten der Epidermis, **wobei** das Verfahren die folgenden Schritte umfasst:
a) optional Reinigen oder Entfernen von Make-up von der Haut;
b) optional Entfetten der Haut, optional auch Durchführen eines chemischen oder mechanischen Peelings;
c) Auftragen einer Zubereitung, die antioxidative Inhaltsstoffe umfasst;
d) Absichern empfindlicher Bereiche wie der Augenwinkel, der Nasenflügel, der Augenbrauenlinien, der Mundwinkel;
e) Auftragen der Zusammensetzung nach einem der Ansprüche 1-3;
wobei die in Schritt e) aufgetragene Zusammensetzung für einen Zeitraum von mindestens 6-10 Stunden nicht von der Haut abgewaschen wird.

5. Verfahren zur nicht-therapeutischen Exfoliation der äußeren Schichten der Epidermis nach Anspruch 4, **wobei** die antioxidativen Inhaltsstoffe der in Schritt c) aufgetragenen Zubereitung Vitamin C und Ferulasäure sind.

6. Verfahren zur nicht-therapeutischen Exfoliation der äußeren Schichten der Epidermis nach Anspruch 4 oder 5, **wobei** das Verfahren von einem erfahrenen Fachmann durchgeführt wird.

7. Verfahren zur nicht-therapeutischen Exfoliation der äußeren Schichten der Epidermis nach einem der Ansprüche 4-6, **wobei** die in Schritt e) aufgetragene Zusammensetzung für einen Zeitraum von 6-10 Stunden nicht von der Haut abgewaschen wird.

8. Verfahren zur nicht-therapeutischen Exfoliation der äußeren Schichten der Epidermis nach einem der Ansprüche 4-7, **wobei** das Verfahren den Schritt a) Reinigen oder Entfernen von Make-up von der Haut und/oder den Schritt b) Entfetten der Haut, optional auch Durchführen eines chemischen oder mechanischen Peelings, umfasst.

## Revendications

1. Composition ayant une base hydroalcoolique, comprenant du rétinol, **dans laquelle** la composition comprend du rétinol à une concentration de 3 - 7 % pds et un ingrédient ayant un nom INCI de Nicotiana Benthamiana Hexapeptide-40 sh-Polypeptide-76.

2. Composition selon la revendication 1, **dans laquelle** la composition comprend du rétinol à une concentration de 3.5 - 5 % pds.

3. Composition selon la revendication 1 ou 2, **dans laquelle** la composition comprend l'ingrédient Nicotiana Benthamiana Hexapeptide-40 sh-Polypeptide-76, en quantité de 0.000002 - 0.00003 % pds.

4. Procédé d'exfoliation non thérapeutique des couches externese de l'épidémie, **ledit** procédé comprend les étapes à:
a) éventuellement nettoyer ou démaquiller la peau ;
b) éventuellement dégraisser la peau, éventuellement également effectuer un peeling chimique ou mécanique ;
c) appliquer une préparation comprenant des ingrédients antioxydants ;
d) protéger les zones sensibles telles que les coins des yeux, les ailes du nez, les lignes des sourcils, les commissures des lèvres ;
e) appliquer la composition telle que définie dans l'une quelconque des revendications 1 à 3,
dans lequel la composition appliquée à l'étape e) n'est pas lavée de la peau pendant une période d'au moins 6-10 heures.

5. Procédé d'exfoliation non thérapeutique des couches externese de l'épidémie selon la revendication 4, **dans lequel** ingrédients antioxydants de la préparation appliquée à l'étape c) sont la vitamine C et l'acide férulique.

6. Procédé d'exfoliation non thérapeutique des couches externese de l'épidémie selon la revendication 4 ou 5, **ledit** procédé est réalisée par un professionnel qualifié.

7. Procédé d'exfoliation non thérapeutique des couches externese de l'épidémie selon l'une quelconque des revendications 4 à 6, **dans lequel** la composition appliquée à l'étape e) ) n'est pas lavée de la peau pendant une période de 6-10 heures.

8. Procédé d'exfoliation non thérapeutique des couches externese de l'épidémie selon l'une quelconque des revendications 4 à 7, **ledit** procédé comprend l'étape a) de nettoyer ou démaquiller la peau et/ou l'étape b) de dégraisser la peau, éventuellement également effectuer un peeling chimique ou mécanique.
